# EUROPEAN PATENT APPLICATION

(11) **EP 2 095 824 A1**
(43) Date of publication of application: **02.09.2009**
(21) Application number: 08003572.8
(22) Date of filing: 27.02.2008
(51) Int. Cl.: A61K 38/45, A61P 9/12, A61P 19/00, A61P 25/00

(54) **A method for restoring BMP-receptor signaling in a cell**

(71) Applicant: Freie Universität Berlin, 14195 Berlin (DE)
(72) Inventor: Knaus, Petra, 14195 Berlin (DE); Schwappacher, Raphaela, 10965 Berlin (DE)
(74) Representative: Engelhard, Markus

(57) **Abstract**

The invention relates to a method for restoring BMP-receptor signaling in a cell. According to the invention, the activity of the protein cGKI is increased in a cell. Furthermore, the invention relates to the use of cGKI for the treatment of a disease selected from the group consisting of pulmonary artery hypertension (PAH), cancer, fibrosis, bone diseases, and neurodegenerative diseases, and the use of cGKI for manufacturing a pharmaceutical composition for the treatment of said diseases, the use of a BMP receptor for screening for compounds having cGKI activity, the use of cGKI for screening for receptors associated with it, and the use of cGKI for the transcriptional activation of genes containing a BMP response element.

## Description

The invention relates to a method for restoring absent or decreased BMP-receptor signaling in a cell, as well as to its use for the treatment of pulmonary artery hypertension. Furthermore, the invention relates to the use of cGKI for manufacturing a pharmaceutical composition for the treatment of pulmonary artery hypertension. In addition, the invention relates to the use of a BMP receptor for screening for compounds having cGKI activity, to the use of cGKI for screening for receptors associated with it, and to the use of cGKI for the transcriptional activation of genes containing a BMP response element.

### Introduction

Bone Morphogenetic Proteins (BMPs) regulate a plethora of cellular processes in embryonic and mature tissue (Canalis et al., 2003; Lories and Luyten, 2005; Schier and Talbot, 2005; Varga and Wrana, 2005). The transduction of BMP signals is fine regulated on each step ranging from controlling the availability of the extracellular ligand to the concert of nuclear factors regulating the transcriptional response triggered by BMPs. The BMP ligand binds two specific transmembrane serine/threonine kinase receptors, BMP type I (BRI) and BMP type II (BRII) receptor. These receptors either reside preassembled in heteromeric complexes (PFC: preformed complex) prior to ligand binding or exist as monomers or homodimers (Gilboa et al., 2000). Ligand binding to PFCs triggers phosphorylation of BRI by BRII, and propagation of the signal by phosphorylation and thereby activation of Smad 1/5/8 (Nohe et al. 2002). The signal is then transduced via heteromeric complex formation between Smad1/5/8 and co-Smad4 which enter the nucleus to regulate BMP-specific target gene expression (Feng and Derynck, 2005; Shi and Massague, 2003). Non-Smad signalling, however, is initiated by binding of BMP-2 to the high affinity receptor BRI, which subsequently recruites BRII to activate the MAPK pathway (Nohe et al. 2002; Canalis et al., 2003).

BMP signaling is fine-tuned at multiple levels, depending on environmental inputs and developmental stage. Ligand accessibility is modulated by antagonists, receptor activation is controlled by co-receptors, by their specific membrane localization and endocytosis, as well as by receptor associated proteins (Feng and Derynck, 2005; Satow et al., 2006) (Hartung et al., 2006). More recently, it was shown that BMP R-Smads are phosphorylated while the activated BMP receptor complex is still at the plasma membrane. Continuation of signaling, i.e. release of Smads from the receptors to translocate into the nucleus, requires clathrin-mediated endocytosis of the receptors (Hartung et al., 2006, MCB).

The detailed mechanism of how this endocytosis is regulated is still not known, but is of special importance for the specificity, intensity and duration of BMP signal transduction. A number of BRII accessory proteins have recently been described as critical regulators of BMP signaling (Chan et al., 2007; Foletta et al., 2003; Lee-Hoeflich et al., 2004; Machado et al., 2003; Wong et al., 2005). Furthermore, the availability of Smads for receiving the signal from the receptors as well as their activity is also modulated by accessory proteins (Reguly and Wrana, 2003; Feng and Derynck, 2005). The dynamic interplay of the Smad pathway with mitogen activated protein kinases (MAPKs) and phosphatases (Sapkota et al., 2007) allows essential fine regulation of this step in signal transduction (Duan et al., 2006, and references therein).

Finally, nuclear BMP signaling depends on cooperation of Smads with proteins of the nuclear envelope like XMAN1 (Osada et al., 2003; Xao et al., 2001) and on recruitment of specific transcriptional factors (Feng and Derynck, 2005) to control nucleo-cytoplasmic shuttling, activity status and DNA binding of Smads. Together, these mechanisms generate feedback loops and, in crosstalk with other signal pathways, prevent malfunctions during signaling by a strict control of every single component.

Two BRII isoforms arise from alternate spliced mRNAs (Rosenzweig et al., 1995). BRII long form (BRII-LF) in contrast to the short form (BRII-SF) exhibits a long cytoplasmic extension (BRII-tail), which is unique among mammalian TGFβ superfamily receptors. Although several studies show equal BMP initiated signaling characteristics for BRII-SF and BRII-LF (Liu et al., 1995; Nohe et al., 2002), signaling functions as well as signaling crosstalk could be attributed to the C-terminal tail of BRII (Chan et al., 2007; Foletta et al., 2003; Hassel et al., 2006; Lee-Hoeflich et al., 2004; Machado et al., 2003; Rudarakanchana et al., 2002; Wong et al., 2005).

Defects in BMP signaling are known to cause diseases, such as pulmonary artery hypertension (PAH).

### Description of the Invention

Accordingly, the problem underlying the present invention was to provide a means for restoring faulty BRII signaling.

This problem is surprisingly solved by the present method for restoring or improving BMP-receptor signaling in a cell. According to the invention, the cGKI activity in a cell is increased. This method can be used e.g. when the BMP/cGKI pathway is interrupted, e.g. due to a mutation in the BRII protein. The increase in cGKI expression compensates for the defect in the signaling cascade and restores the signaling pathway. An advantage of this method is that only the relatively small molecule cGKI needs to be provided, and not, e.g. the large transmembrane protein BRII.

cGKI is composed of three functional domains: an N-terminal domain, which is encoded by an alternatively spliced exon generating cGKIα and P isoforms, a regulatory domain, and a catalytic serine/threonine kinase domain (Feil et al., 2005; Lohmann and Walter, 2005; Pilz and Broderick, 2005). cGKI as used herein is meant to refer to either cGKIα or cGKIβ, or both isoforms of the enzyme, if not specified otherwise.

The term "cGKI activity" refers to the overall enzymatic activity of the protein cGKI in the cell to be restored.

According to the invention, the increase of the cGKI activity in a cell can be achieved by at least one of several different means. In one preferred embodiment of the invention, the increase of the cGKI activity is achieved by overexpressing a polypeptide selected from the group consisting of:
- a polypeptide that comprises or is identical to cGKI according to SEQ ID NO 1 (α isoform) or SEQ ID NO 2 (β isoform);
- a polypeptide comprising the kinase domain of cGKI (amino acids (aa) 359 to 619 of SEQ ID NO 1; aa 374 to 634 of SEQ ID NO 2) and the "peptide binding domain" of cGKI (from about aa 476 (for the α isoform) or aa 491 (for the β isoform), to the C terminus (aa 671 (for the α isoform) or aa 686 (for the β isoform), respectively); and
- a polypeptide containing a portion of the sequence according to SEQ ID NO 1 or SEQ ID NO 2, that exhibits cGKI signaling function and is able to restore or at least partially restore BMP-receptor signaling in a cell in the absence of a functional BRII-receptor; and
- a polypeptide that is at least 80 % homologous, preferably 90 %, 95 % or, most preferably 99 % homologous to a polypeptide as mentioned above.

The term "homologous" is used here to refer to the similarity in a protein sequence based on the physicochemical nature of the amino acid the protein consists of at a given position. In order to determine homology of two protein sequences to each other, a person of skill in the art may use a computer program, such as BLAST (Basic Local Alignment Search Tool).

The autoinhibitory/dimerization region of cGKI is located from a 1 to 89 (α isoform) and from aa 1 to 104 (β isoform). The cGMP binding regions are from aa 103 to 212 and from 222 to 226 (α isoform) and from aa 118 to 227 and from aa 237 to 341 (β isoform). The Ser/Thr kinase region is located from aa 359 to 619 (α isoform) and from aa 374 to 634 (β isoform).

The overexpression of such a polypeptide can be achieved by transfecting the cell with a polynucleotide encoding for a polypeptide as mentioned above, or using electroporation or injection. The polynucleotide can e.g. be in the form of an expression vector such as a plasmid. Ways of transfecting a cell are known to a person of skill in the art.

Another means of increasing cGKI activity in a cell is to introduce cGKI protein into the cell, e.g. using transfection, electroporation, transfer using packaging material like micelles, injection or combinations thereof.

In another preferred embodiment of the invention, the increase of the cGKI activity is achieved by expressing a constitutively active form of cGKI in a cell. Such a constitutively active form of cGKI is generated in general terms by mutating the autoinhibitory site such that it cannot fold into the active site of the kinase domain. Thereby, the ability of cGKI for autoinhibition is abolished, and the kinase domain of cGKI is in a constantly activated mode. A person of skill in the art will be able to generate such a constitutively active form of cGKI based on the information given here together with his general knowledge. Preferably, such a constitutively active form of cGKI α isoform bears a mutation from the group consisting of aa 1-78 delta, 1-325 delta, Thr58Glu and Ser64Asp. A constitutively active form of the cGKI β isoform bears a mutation from the group consisting of aa 1-92 delta, 1-340 delta, and Ser79Asp. The point mutant cGKI β Ser79Asp is preferred, since a point mutation effects the conformation of the entire protein less than a deletion.

In yet another preferred embodiment of the invention, the increase of the cGKI activity is achieved by inactivating a protein that inhibits cGKI activity. An example for such an inhibitor of cGKI activity is phosphodiesterase-5 (PDE5), which degrades cGMP in the cell. Known inhibitors of PDE5 that can be used to increase the activity of cGKI are e.g. sildenafil (Viagra^{™}), tadalafil and/or vardenafil.

It is preferred that the increase in cGKI activity is accompanied by the addition of a BMP-receptor ligand to the cell whose BMP-receptor signaling is to be restored or improved. Thereby, the BMP signaling pathway is triggered by a natural or artificial ligand as well as by the action of the cGKI kinase that is present in the cell either in a higher concentration than in a wild type cell or in a constitutively active mutant form. Such a ligand can be BMP-2, BMP-7 and/or GDF-5, or any mutant of such a ligand. It will be understood by a skilled person that this approach will only increase the action of cGKI in the cell if the BRII receptor is at least partially functional and, upon BMP-2 binding, transduces a (in comparison to a wild type BRII receptor maybe weaker) signal into the cell.

The invention can be performed with a cell from a mammal, preferably from mouse or a human. Ways of obtaining the proteins and/or nucleic acids of interest for a given species are known to a person of skill in the art. In one embodiment of the invention, the method is performed ex vivo.

The invention also pertains to a cell with increased cGKI activity. Such a cell can be obtained by performing a method as described above.

The problem underlying the present invention is also solved by the use of a BMP receptor for screening for compounds having cGKI activity or cGKI-like activity. As the BMP receptor, both BRII and/or BRI can be used.

In this use, it is preferred that a BMP receptor protein is isolated from a cell under conditions that allow for the co-isolation of a protein that is functionally associated with the BMP receptor protein in the cell. In order to achieve this co-isolation, a tagged version of the BMP receptor protein is preferably expressed in the cell. As a tag, GST- and/or HA-tag can be used encoded on an expression vector to express a BMP receptor fusion protein. An (immuno) precipitation assay using an antibody or a tag binding protein can for example be used to identify the protein associated with it. The identification can be achieved by methods like Western blot, sequencing, MALDI-TOF, or other methods known to a person of skill in the art.

The protein associated with a BMP receptor might exhibit a function similar to cGKI in the cell and is therefore tested for exhibiting cGKI function. A protein exhibiting such cGKI function can also be used to overcome an ill-functioning BMP receptor.

The use of cGKI for screening for receptors associated with it also solves the problem underlying the present invention. Thereby, a cGKI protein is isolated from a cell under conditions that allow for the co-isolation of a membrane protein that is functionally associated with the cGKI protein in the cell. The expression of tagged cGKI protein in the cell is preferred, e.g. using a GST-/HA-cGKI fusion construct. Following precipitation and/or immuno precipitation using an antibody or a tag binding protein, the membrane protein associated with cGKI can be identified, e.g. through Western blot, sequencing, MALDI-TOF, etc. The identified receptor can then be used as an alternative means of activating the cGKI pathway and thereby overcome faulty BMP-signaling of the cell.

The problem underlying the present invention is also solved by the use of cGKI for the transcriptional activation of genes containing at least one BMP response element (BRE). Such genes are the target or effector of the BMP-signaling pathway.

The inventors have found that upon cGKI activation, a transcriptional activation complex is formed consisting of several proteins. Such a transcriptional activation complex comprises or consists of cGKI, together with Smad proteins (1, 5, 8, 4), and/or TF-II. This transcriptional activation complex translocates into the nucleus where it binds to promoter regions containing a BRE. Thereby, transcription of the gene under the control of the BRE containing promoter is induced and/or enhanced. Therefore, the increase in activity leads to the transcriptional activation of genes containing BRE elements. An example of such a gene is Id1. BRE elements are also common in the promoter/regulatory regions of transcription factors, e.g. transcription factors of the Wnt family, and known to be present in the osterix gene.

Preferably, cGKI is used to activate a gene that comprises a so-called "cGKI response element" in addition to at least one BRE in its promoter region or regulatory region. An example for a gene with such a "cGKI response element" is the Egr1 gene, whose promoter region comprises a BRE and a "cGKI response element", as shown in the examples.

In another embodiment, cGKI is used for the treatment of pulmonary artery hypertension (PAH) (increasing muscle relaxation) or by the use of the method for restoring BMP-receptor signaling in a cell as described above.

Pulmonary arterial hypertension (PAH) results from the tightening or blockage of blood vessels to and within the lungs. As increasing numbers of vessels become blocked, blood flow through the lungs is impeded. The right ventricle of the heart compensates by generating higher pressure. As the blood flowing through the lungs decreases, the left side of the heart receives less blood. This blood may also carry less oxygen than normal. Therefore, it becomes increasingly difficult for the left side of the heart to supply sufficient oxygen to the rest of the body, especially during physical exertion. Finally, when the right ventricle can no longer compensate, heart failure ensues.

The gene that has been linked to familial form of PAH is BR II (BMPR-2). Previous analysis (Foletta et al, 2003) has shown that BRII binds to LIMK1, a protein responsible for phosphorylating cofilin. The addition of a ligand, BMP 4, inhibits the phosphorylation of cofilin by LIMK1. Truncations in the C-terminal domain of BR II that prevent the binding of LIMK1 also prevent the inhibition of LIMKI. This was the first study that linked mutations in the tail region of BR II with the deregulation of actin dynamics in the etiology of BR II-related PAH.

As recently as 2005, sildenafil, a selective inhibitor of cGMP specific phosphodiesterase type 5 (PDE5), was approved for the treatment of PAH. The present invention elucidates the mechanism for this empirically found treatment.

It was now surprisingly found by the inventors that PAH can be treated by increasing the cGKI activity in a cell. This increase of activity can be achieved through various means, as described above, including overexpression of the cGKI protein or use of a constitutively active cGKI.

Accordingly, in another aspect of the invention, cGKI is used for treating of pulmonary artery hypertension (PAH). Furthermore, in yet another aspect of the invention, cGKI is used for the treatment of cancer, fibrosis, bone diseases, including brachydaktyly and fractures (in particular non-healing or slow healing fractures), and neurodegenerative diseases.

In another embodiment, cGKI is used for manufacturing a pharmaceutical composition for the treatment of a disease from the group consisting of pulmonary artery hypertension, cancer, fibrosis, bone diseases, including brachydaktyly and fractures (in particular non-healing or slow healing fractures), and neurodegenerative diseases, and/or for treating a disease from said group.

The pharmaceutical composition can either comprise or contain cGKI or a polypeptide derived therefrom, and/or a nucleic acid that allows for the expression of cGKI or a polypeptide derived therefrom, in particular a polypeptide as described above. Such a nucleic acid can be a DNA, a cDNA, a RNA molecule, or derivatives therefrom, in particular a nucleic acid that enocodes for a polypeptide as referred to above. For delivery of such polypeptides or nucleic acids, the pharmaceutical composition can comprise or contain e.g. a virus or a liposome for delivery of the polypeptide or the polynucleotide into a cell. Ways of producing such a pharmaceutical composition are known to a person of skill in the art.

cGKI can be used to treat a cell, a tissue or an organisms with a disease or a condition that is associated with impeded or interrupted BMP-receptor signaling, insofar as the activity of cGKI is increased in the cell, tissue or organisms to be treated. Diseases associated with such an impeded or interrupted BMP-receptor signaling are selected from the group consisting of PAH, cancer, fibrosis, bone diseases, including brachydaktyly and fractures (in particular non-healing or slow healing fractures), and neurodegenerative diseases.

### Figures

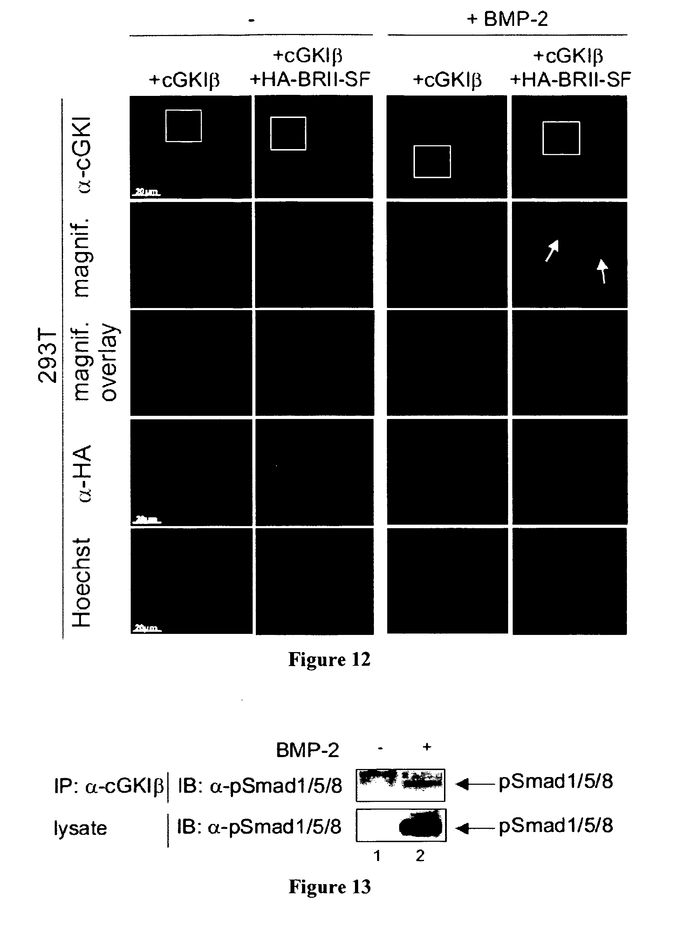

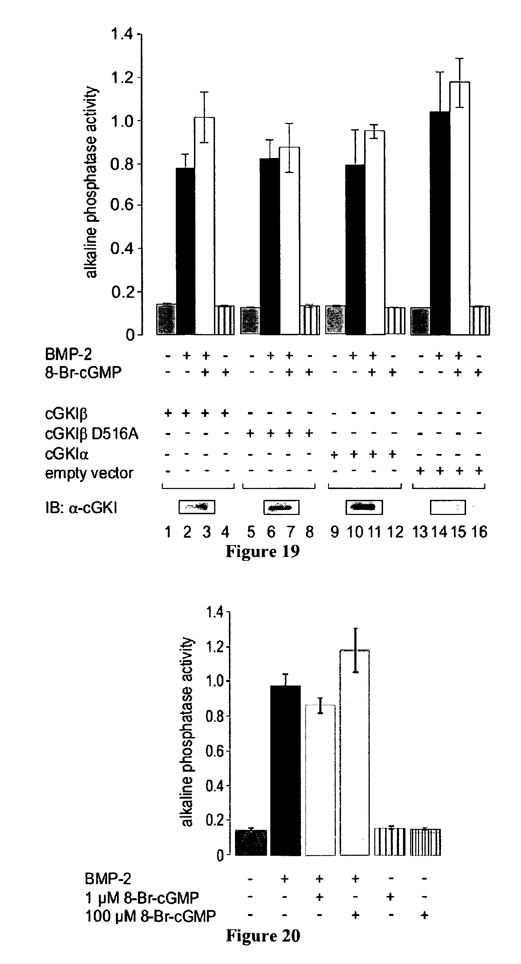

The invention is now described with reference to the figures.

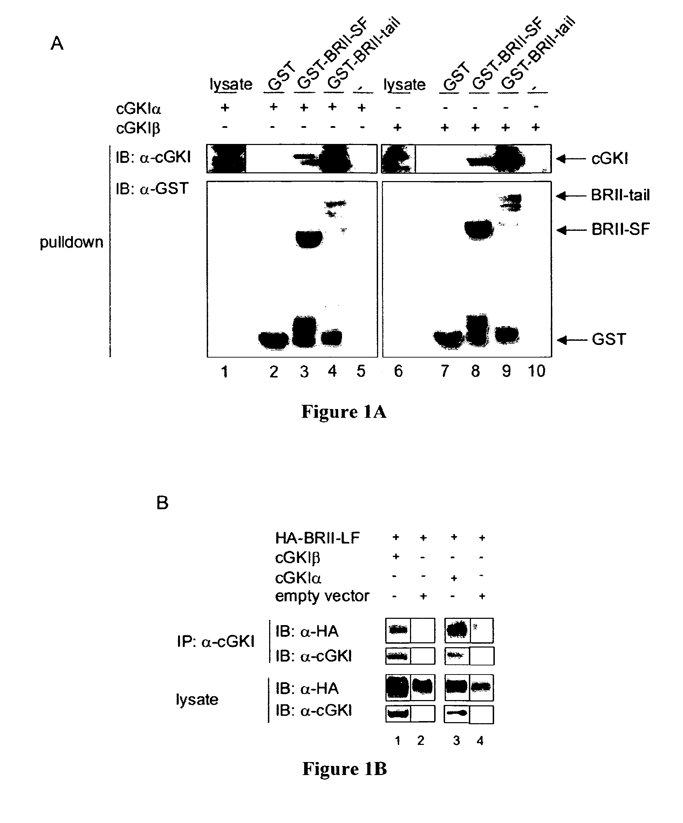

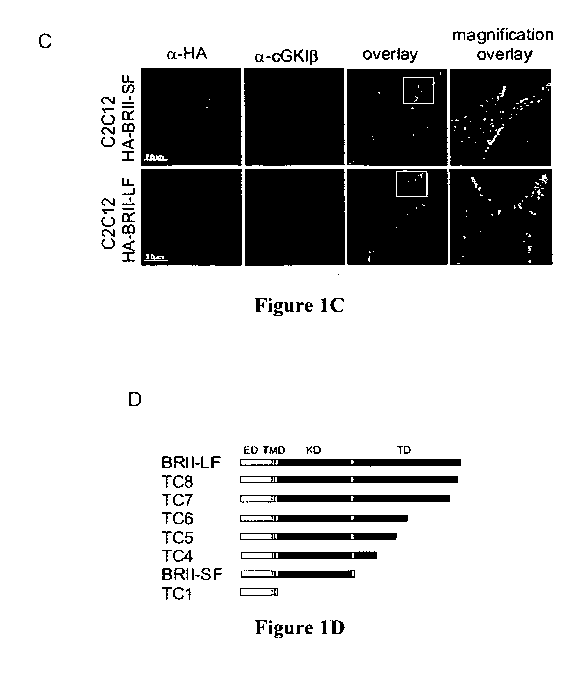

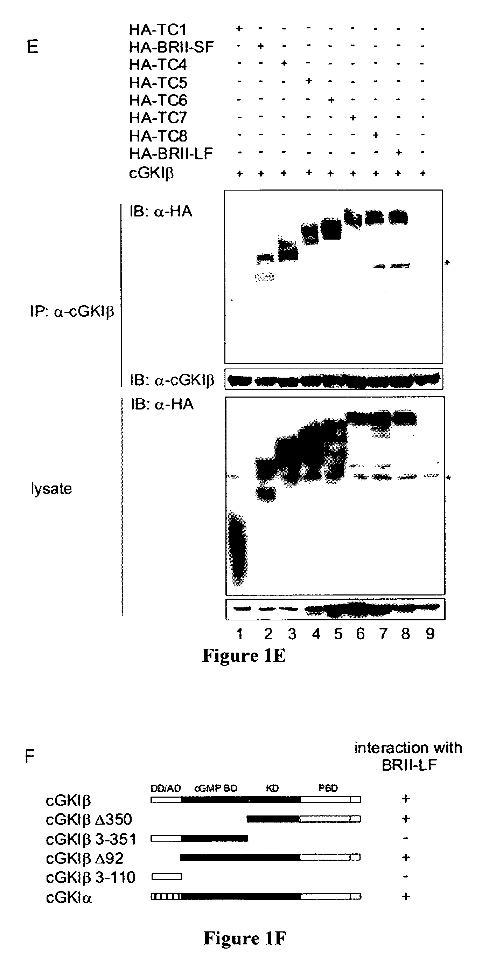

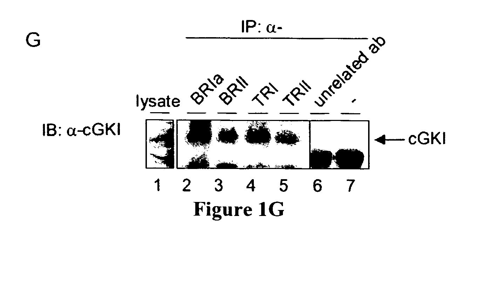

**Figure 1****. Identification of cGKI as a BRII-associated protein** (A) GST-BRII-SF (includes the complete kinase domain (leucine 175 to arginine 530)) and GST-BRII-tail (methionine 501 to leucine 1038) immobilized to glutathione sepharose beads were incubated with C2C12 lysates expressing the indicated cGKI isoform. Purified protein complexes and cGKIα/β expression were examined by immunoblotting with α-cGKI antibody. The input of BRII fusion proteins was visualized using α-GST antibody. (B) 293T cells were transfected with cGKIαor β and HA-BRII-LF. The α-cGKI immunoprecipitates were examined by immunoblotting using α-HA antibody. Lysates were used for control of protein expression. (C) Co-localization of BRII and cGKIβ was analyzed by confocal immunofluorescence microscopy in C2C12 cells stably expressing N-terminally HA-tagged BRII after receptor co-patching. (D) Schema of BRII truncation mutants, ED extracellular domain (white), TMD transmembrane domain (light grey), KD kinase domain (black), TD tail domain (grey). (E) 293T cells were transfected with cGKIβ and N-terminally HA-tagged truncation mutants of BRII. Immunoprecipitates were examined by immunoblotting using α-HA and α-cGKIβ antibodies. Lysates were used to monitor the expression of BRII truncations and cGKIβ. (F) Schema of GST-cGKI fusion proteins used to study cGKI/BRII interaction. Lysates of 293T cells expressing HA-BRII-LF and GST-cGKI truncations were subjected to IP or pulldown assay to analyze cGKI/BRII interaction. The analyzed data are summarized in the list. DD/AD, dimerization domain/autoinhibitory domain (white (cGKIβ), striped (cGKIα)); cGMP BD, cGMP binding domain (black); KD, kinase domain (grey); PBD, peptide binding domain (white) (G) Endogenous complexes containing cGKI in C2C12 cells were analyzed by IP with α-BRIa, α-BRII, α-TRI and α-TRII antibodies and subsequent immunoblotting with α-cGKI antibody. IB, immunoblotting; IP, immunoprecipitation.

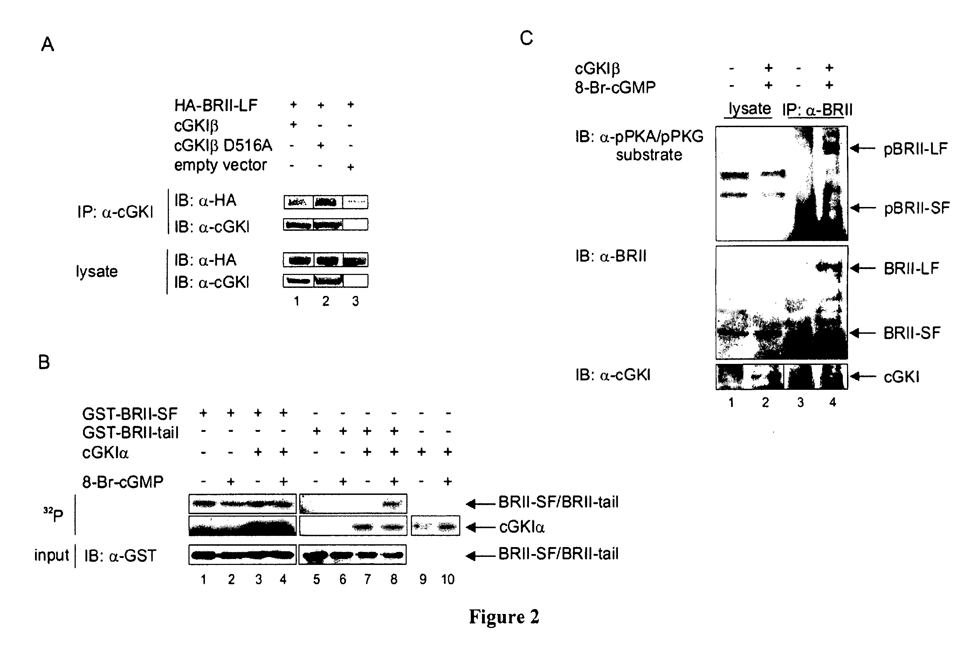

**Figure 2****. cGKI and BRII interaction is independent of both kinase activities, but cGKI phosphorylates BRII** (A) From 293T cell lysates co-expressing BRII-LF and cGKI variants cGKI was immunoprecipitated. Precipitates and lysates were analyzed using α-HA and α-cGKI antibody. (B) GST-BRII-SF and GST-BRII-tail immobilized to glutathione sepharose beads were subjected to *in vitro* kinase assay with cGKIα, activated or not using 8-Br-cGMP. Incorporated ³²P was detected by autoradiography. Input of fusion proteins was visualized by immunoblotting using α-GST antibody. (C) Endogenous BRII, enriched via IP, was analyzed under the influence of cGKIβ using a pPKA/PKG substrate specific antibody. Protein expression was monitored with α-BRII and α-cGKI antibody.

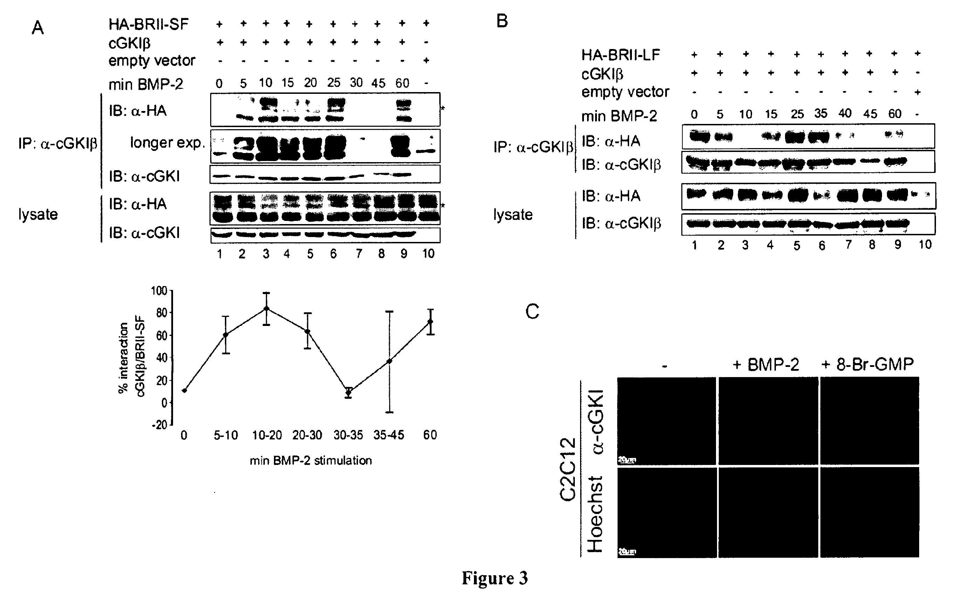

**Figure 3****. cGKI detaches from the receptor complex and undergoes nuclear translocation upon BMP-2 stimulation** (A and B) 293T cells were co-transfected with cGKIβ and HA-tagged BRII-SF (A) or HA-BRII-LF (B). Cells were stimulated with BMP-2 from 5 to 60 min. cGKIβ was immunoprecipitated from cell lysates followed by immunoblot using α-HA antibody. Protein expression was controlled by immunoblotting with α-cGKI and α-HA antibodies. These experiments are exemplarily (n>3). Results for cGKIβ/BRII-SF were quantified using ImageJ and error bars represent deviation of the mean of two experiments. (C) Immunofluorescence staining of cGKI in C2C12 cells after stimulation with BMP-2 or 8-Br-cGMP. DNA was stained using Hoechst dye.

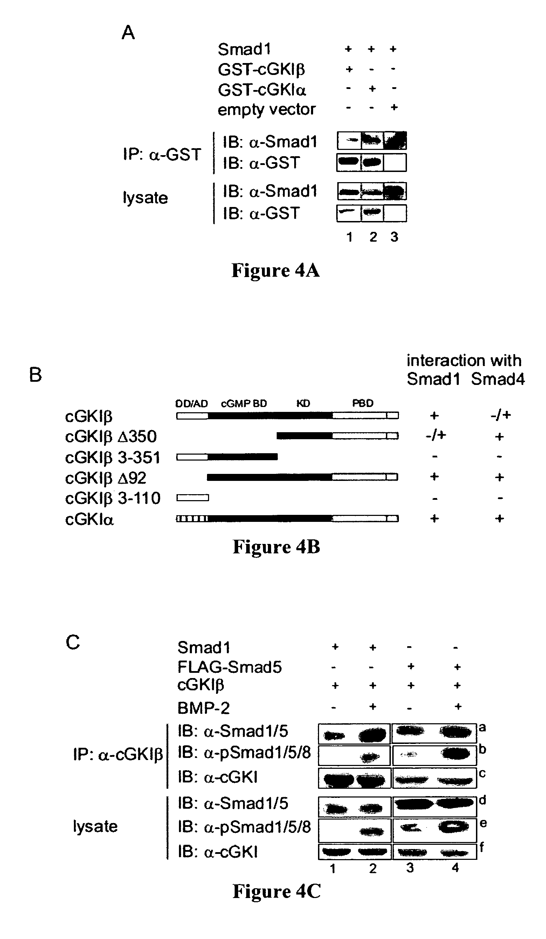

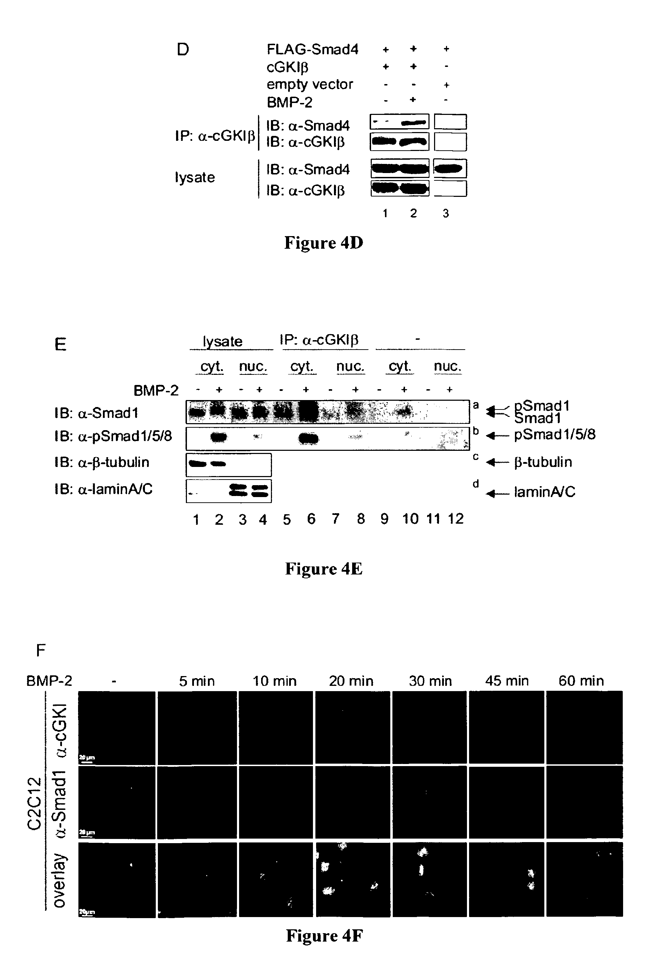

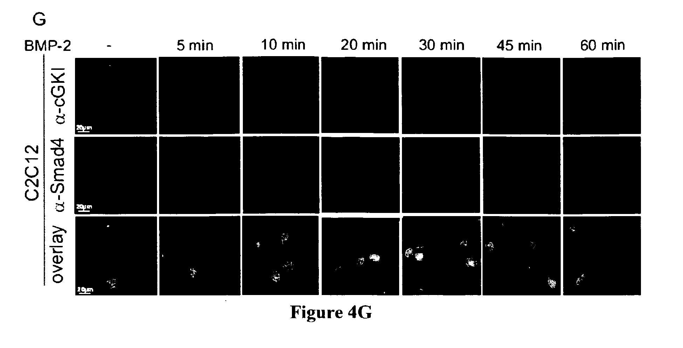

**Figure 4****. cGKI associates with Smad complexes** (A) Binding of GST-fused cGKIα or β to Smad1 in 293T cells. (B) Schema of GST-cGKI fusion proteins used to study cGKI/Smad interaction, as in Figure 2C. Lysates of 293T cells expressing Smad1 or Smad4 and GST-cGKI truncations were subjected to IP or pulldown assay to analyze cGKI/BRII interaction. The analyzed data are summarized in the list. (C) 293T cells were co-transfected with Smad1 or FLAG-Smad5 and cGKIβ and stimulated with BMP-2 or left untreated. cGKIβ immunoprecipitates were subjected to immunoblotting using α-Smad1/5, α-P-Smad1/5/8 and α-cGKI antibodies. Levels of pSmad1/5/8, total Smad and cGKIβ were detected by immunoblotting. (D) As in (C), except that cells were co-transfected with FLAG-Smad4 and cGKIβ and Smad4 was assayed for interaction with cGKIβ. Immunoprecipitates and control lysates were analyzed using α-Smad4 and α-cGKIβ antibodies. (E) C2C12 cells were stimulated with BMP-2 or left untreated and were subjected to cytoplasmic-nuclear fractionation. Both lysed cell fractions were incubated with an antibody directed towards cGKI for IP. Precipitates and lysates were analyzed using α-Smad1 and α-pSmad1/5/8 antibodies. To control fractionation lysates were probed with α-β-tubulin and α-laminA/C antibodies. (F and G) C2C 12 cells were starved and stimulated with 10 nM BMP-2 (5 to 60 min) of left untreated (-). Cells were co-stained for intracellular cGKI and Smad1 (F) or Smad4 (G) using specific antibodies. Lower panels monitor the co-localization by overlay.

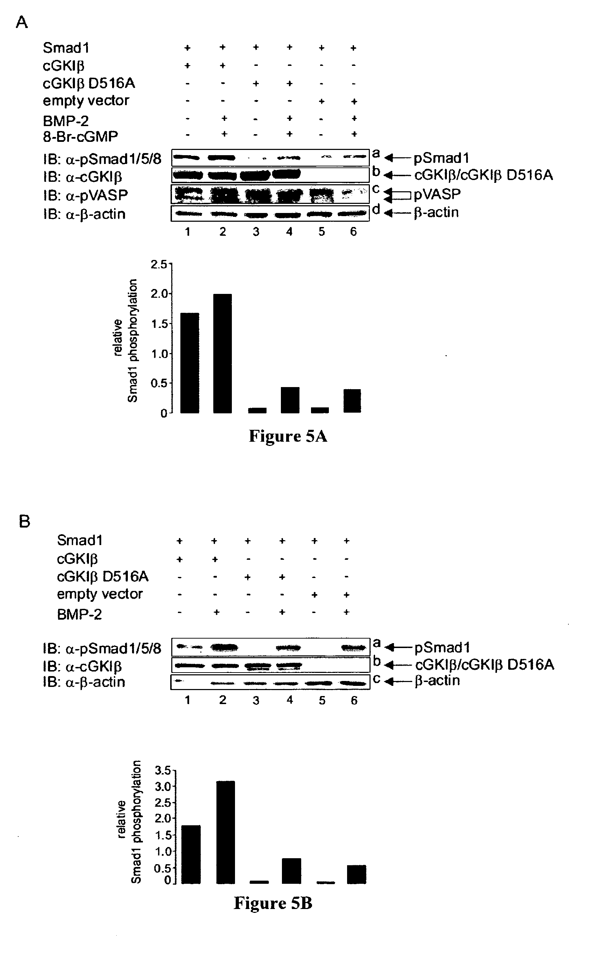

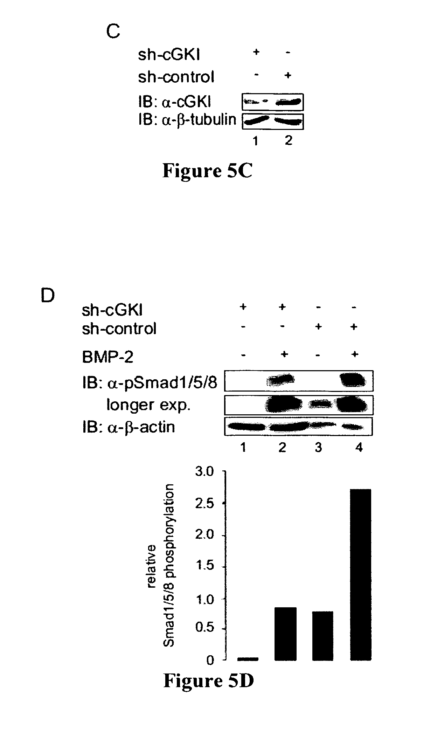

**Figure 5****. cGKI stimulates R-Smad phosphorylation at the C-terminus** (A) 293T cells transfected with cGKIβ, cGKIβ D516A mutant and Smad1 were stimulated with BMP-2 and 8-Br-cGMP or left untreated. Whole cellular extracts were subjected to immunoblot using α-pSmad1/5/8 antibody. α-cGKIβ and α-β-actin immunoblotting was used as expression and loading control. To monitor the activity of cGKIβ the membrane was re-probed with α-pVASP antibody. Results were quantified using ImageJ and Smad1 C-terminal phosphorylation was normalized relative to β-actin. (B) As in (A) except that 293T cells were stimulated with BMP-2 without 8-Br-cGMP. (C) Efficiency of cGKI knock down using a specific short hairpin-RNA (sh-cGKI) compared to a control (sh-control) was validated in C2C12 cells via immunoblot using α-cGKI antibody. Applied protein amount was controlled by α-β-tubulin antibody. (D) C2C12 cells were transfected with sh-cGKI and sh-control and subjected as in (A) to Smad phosphorylation assay. Smad1/5/8 phosphorylation and protein loading was monitored with α-pSmad1/5/8 and α-β-actin antibodies. Results were quantified using ImageJ and Smad1/5 C-terminal phosphorylation was normalized relative to β-actin.

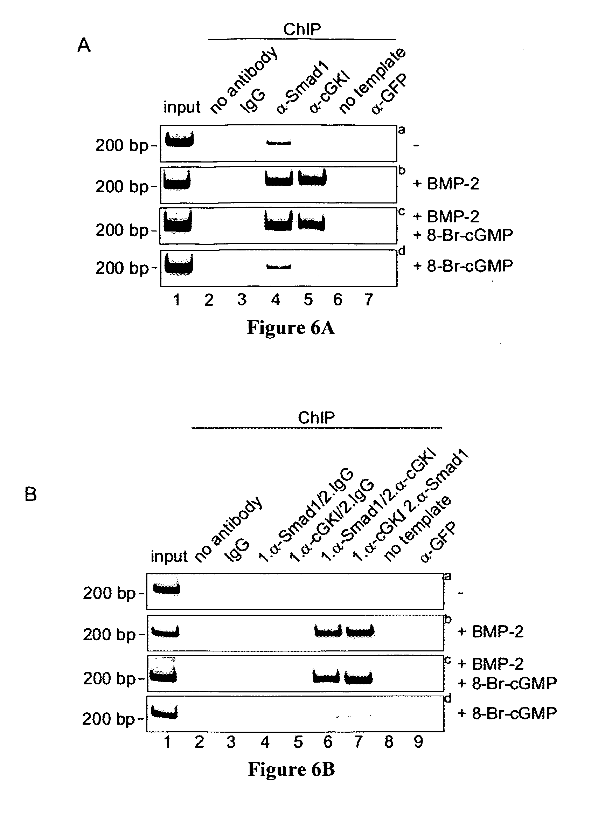

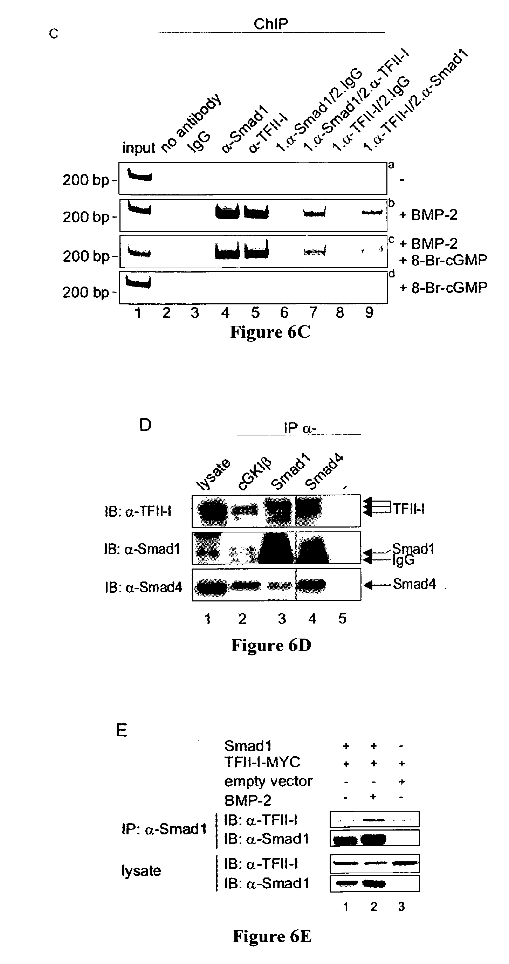

**Figure 6****. cGKI, Smad1 and TFII-I co-localize at the *Id1* promoter after stimulation with BMP-2** (A, B and C) C2C12 cells were stimulated with BMP-2 and/or 8-Br-cGMP or left unstimulated (-). Chromatin immunoprecipitation (ChIP) was performed using (A) α-cGKI or α-Smad1 antibody or (B) α-Smad1 followed by α-cGKI antibody and vice versa or (C) α-Smad1 followed by α-TFII-I antibody and vice versa. Subsequent PCR identified a co-precipitated *Id1* promoter sequence using specific primers. The applied amount of DNA for ChIP is shown in lane 1. To exclude unspecific outcome control samples were run (for ChIP: no antibody, IgG, α-GFP antibody; for two-step ChIP: α-respective antibody followed by IgG; for unspecific amplification via PCR: no template). (D) Endogenous complexes with cGKI from C2C12 cells were analyzed with α-TFII-I, α-Smad1 and α-Smad4 antibodies and a lysate aliquot was monitored for protein expression. (E) Smad1/TFII-I association was analyzed in 293T cells expressing both proteins. IP was performed using a Smad1 directed antibody and the pellets were examined for co-IP of TFII-I. Lysate control was done by immunoblotting α-Smad1 and α-TFII-I.

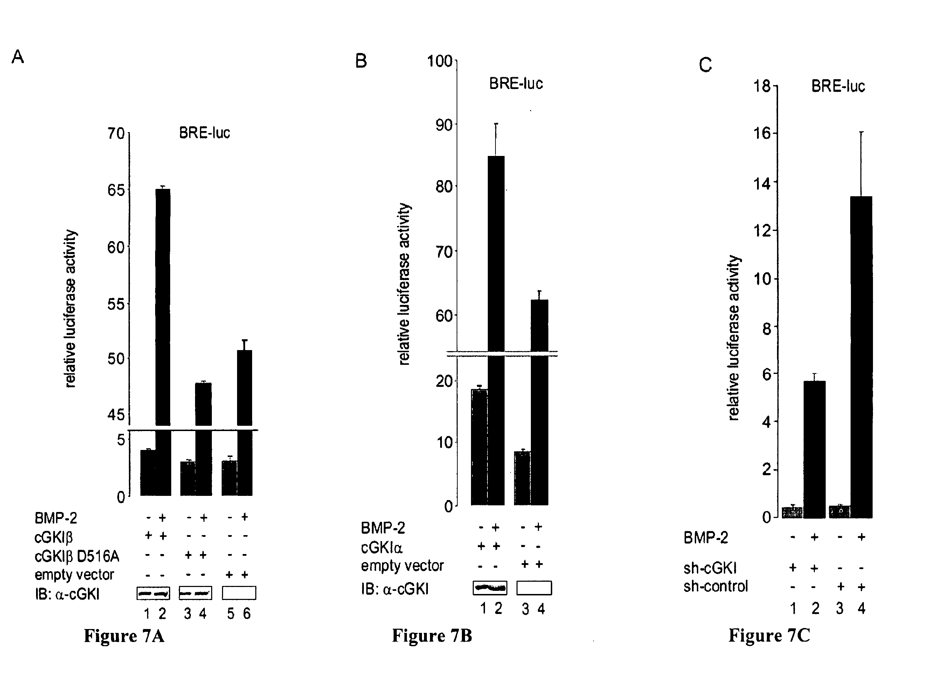

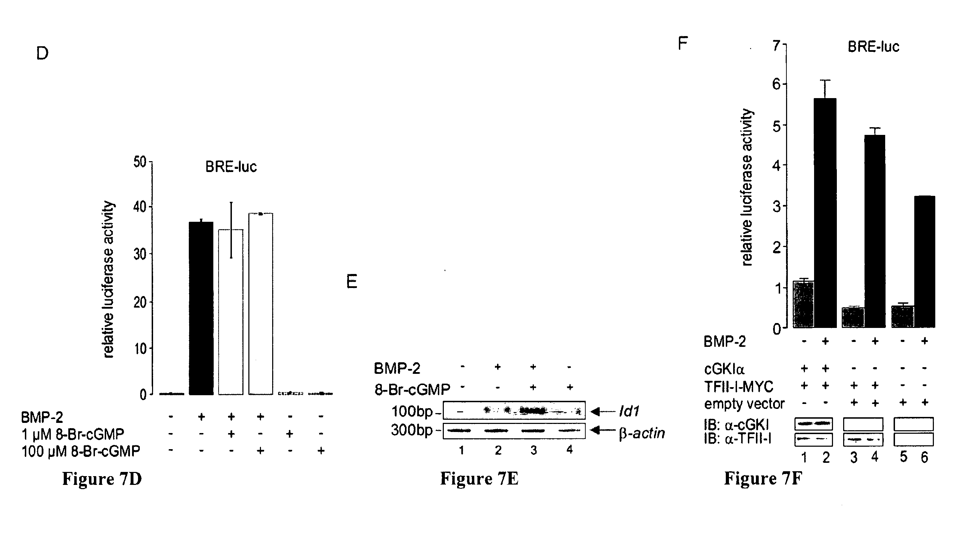

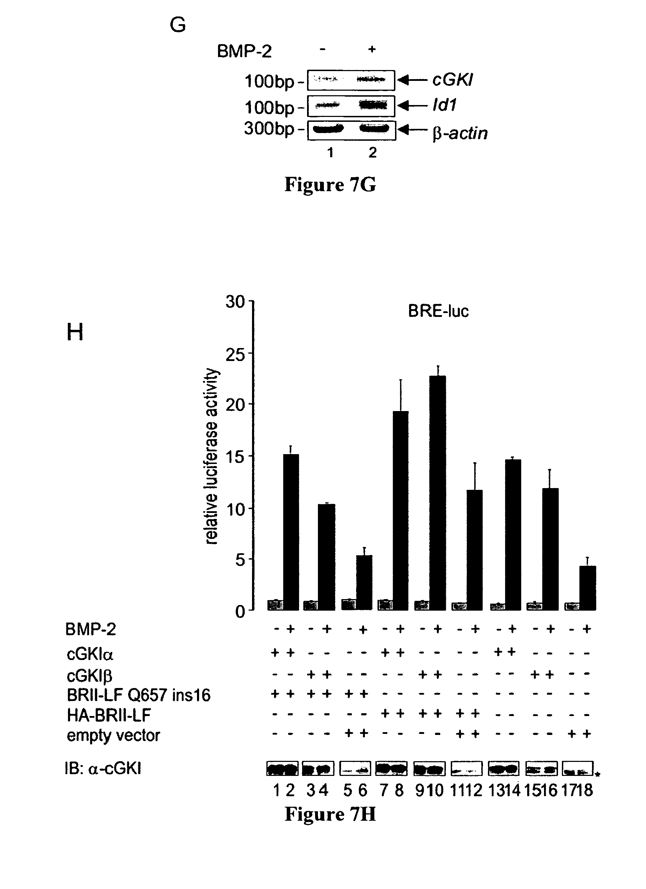

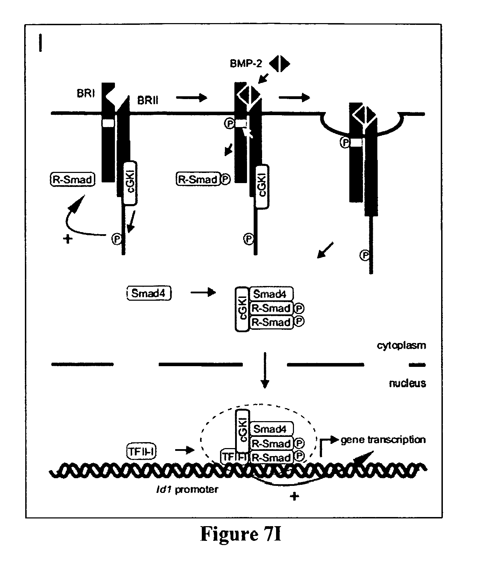

**Figure 7****. cGMP/cGKI pathway stimulates BMP-2 signaling via Smad1/5** (A and B) C2C12 cells were co-transfected with p*BRE*-luc, pRL-TK and cGKI variants and stimulated with BMP-2 or left untreated. Luciferase activity was measured and error bars result from the mean of duplicate measurements. These results were reproduced in three independent experiments. Expression of cGKIα/β was controlled by immunoblot using α-cGKI antibody. (C) As in (A and B) except that reporters were co-transfected with sh-cGKI, sh-cGKI mix and sh-control into C2C12 cells. *BRE* driven luciferase activity was measured and error bars represent deviation of the mean of duplicate measurements. This result is representative for three independent experiments. (D) C2C12 cells were transfected with *BRE*-luc and pRL-TK and stimulated with BMP-2 and/or 8-Br-cGMP. Luciferase activities are represented as mean of each value and standard deviations result from duplicate measurements. (E) C2C12 cells were treated with BMP-2 and/or 8-Br-cGMP or left untreated. Following cell extraction RNA was reverse transcribed into cDNA. The mRNA amount of *Id1* was analyzed using *Id1* specific primers including the control of applied cDNA by PCR using β-*actin* specific primers. The result is derived from a representative experiment. (F) As in (A and B), except that reporters were co-transfected with cGKIα and MYC-TFII-I into C2C12 cells. *BRE* driven luciferase activity was measured, standard deviations result from duplicate measurements. These data were reproduced in three independent experiments. Lysates were pooled and immunoblotted for expression of cGKIα and TFII-I. (G) As in (E) except that mRNA amount of *cGKI* was analyzed using *cGKI* specific primers. The result is derived from an exemplary experiment. Results were quantified using ImageJ. *cGKI* mRNA expression was normalized relative to β-*actin*. (H) As in (A and B), except that the HA-tagged BRII mutant Q567 ins 16 causing sporadic PAH was co-transfected with cGKIβ. Error bars results from the mean of duplicate measurements. This result is representative for three independent experiments. α-cGKI antibody controlled cGKIβ expression. (I) Model: Schematic representation of cGKI interference with BMP signaling indicating the bi-functionality of cGKI through (a) modulation of BMP receptor activity at the cell surface to enhance Smad phosphorylation and association with activated Smad complexes to translocate into the nucleus and (b) regulation of Smad-mediated transcription activation as a nuclear co-factor.
**Figure 8**
   cGKI/BRII-tail complexes in C2C12 cells were isolated using GST-BRII-tail (methionine 501 to leucine 1038) for pulldown and identified by subsequent two dimensional gelelectrophoresis and MALDI-TOF mass spectrometry analysis. The table depicts the proteomics data.
**Figure 9**
   The alignment using ClustalW shows that murine cGKIα and β exclusively differ in their N-terminal part. Peptides identified via Maldi-TOF MS are designated.
**Figure 10**
   Purified proteins, immobilized to glutathion sepharose (GST, GST-BRII-SF and GST-BRII-tail) were analyzed via Coomassie staining. Fusion proteins were subjected to pulldown and *in vitro* kinase assay.
**Figure 11**
   cGKIβ, BRII-SF or BRII-SF K230R kinase deficient proteins, overexpressed in 293T cells, were immunoprecipitated using specific antibodies (α-HA, α-His₆, α-cGKIβ). Proteins precipitated with Protein A sepharose beads were subjected to *in vitro* kinase assay in the presence or absence of 25 µmol/l 8-Br-cGMP. After SDS-PAGE and protein transfer to nitrocellulose membrane, incorporated ³²P was detected by autoradiography. BRII-SF and cGKIβ input was visualized by immunoblot using α-HA, α-His₆ and α-cGKIβ antibodies.
**Figure 12**
   Immunofluorescence labeling of 293T cells expressing cGKIβ with or without HA-BRII-SF was performed using α-cGKI and α-HA antibody after stimulation with BMP-2. Nuclei staining was carried out using Hoechst dye. Nuclear translocation was quantified using ImageJ. Graph shows relative nuclear translocation as measured by "-, +cGKIβ", with values and error bars representing mean and standard deviation of all transfected cells.
**Figure 13**
   Endogenous cGKIβ and Smad1/5/8 form complexes in whole lysates of C2C12 cells after stimulated with BMP-2 as shown by co-IP experiments with α-cGKIβ antibody. Binding of activated Smad1/5/8 was visualized by immunoblotting using α-pSmad1/5/8 antibody. Lysate was controlled by α-pSmad1/5/8 antibody.
**Figure 14**
   As in Figure 13, except that endogenous BMP-2 induced cGKIβ/Smad4 complex formation in C2C12 cells was assayed in a co-IP experiment.
**Figure 15**
   Endogenous VASP phosphorylation in C2C12 cells was monitored in response to 1 µmol/l and 100 µmol/l 8-Br-cGMP stimulation. Lysates were analyzed using immunoblot α-pVASP and α-β-actin.
**Figure 16**
   C2C12 cells were transfected with cGKIβ construct and endogenous Smad1/5/8 phosphorylation was measured using a p-Smad1/5/8 specific antibody. Results were quantified using ImageJ and Smad phosphorylation was normalized relative to β-actin.
**Figure 17**
   For immunofluorescence labeling C2C12 cells were stimulated with BMP-2 or left untreated and co-immunostained for Smad1 and TFII-I. Lower panels show the overlay.
**Figure 18**
   As Figure 7D, except that C2C12 cells were incubated for 24 hrs with BMP-2 and *ALP* mRNA amount was assayed using specific primers.
**Figure 19**
   cGKI transfected C2C12 cells were treated with BMP-2 and/or 8-Br-cGMP and ALP activity was measured. Error bars results from the mean of triplicate measurement and this result was reproduced three times. Pooled lysates were assayed for cGKIα/β expression using α-cGKI antibody.
**Figure 20**
   ALP activity measurement in C2C12 cells was carried out without cGKI overexpression as described in Figure 19. In addition to BMP-2 cells were treated with 1 µmol/l or 100 µmol/l 8-Br-cGMP.
**Figure 21**
   C2C12 cells were transfected with cGKIβ and kinase inactive cGKIβ D516A whose expression was detected with α-cGKI antibody after immunoblot α-pp38. Stimulation was carried out for 5 hrs with BMP-2/8-Br-cGMP.
**Figure 22**
   C2C12 cells were stimulated with BMP-2 and/or 8-Br-cGMP or left untreated. Whole cell lysates were examined by immunoblotting α-pp38 and α-β-actin as loading control.

### Examples

### cGKI interacts with BRII

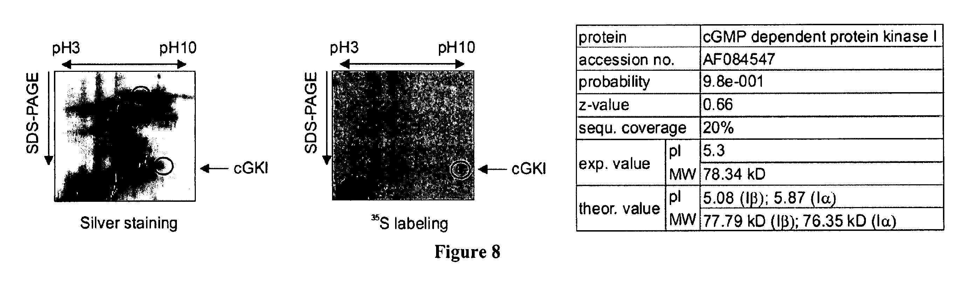

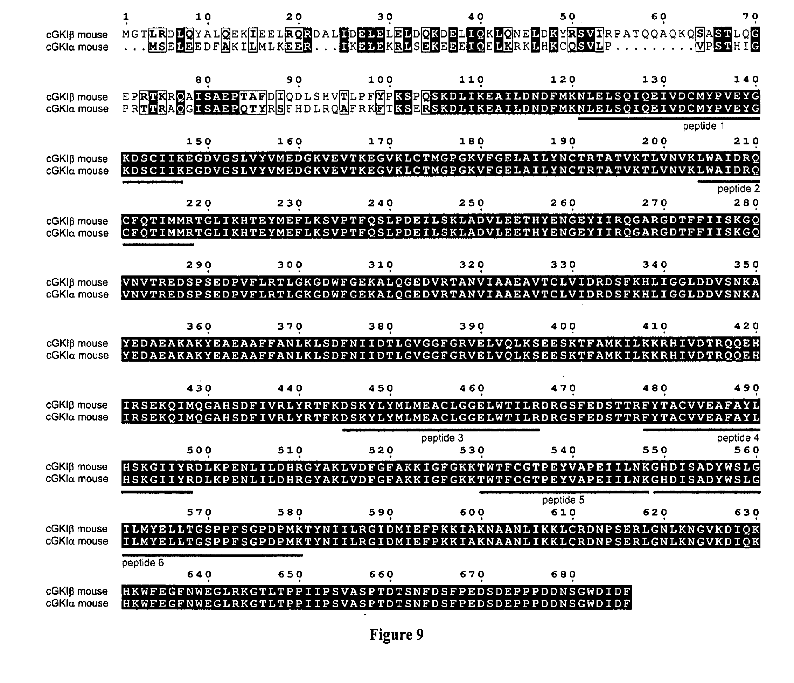

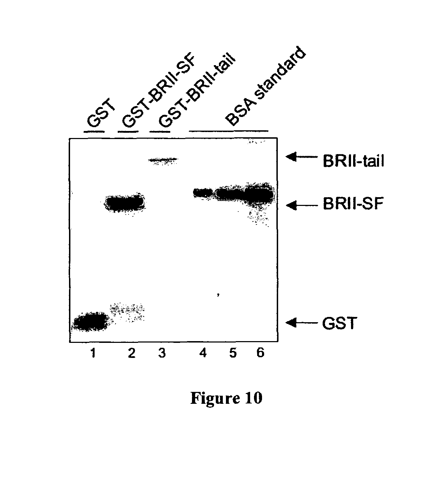

To identify new proteins that regulate BMP signaling, GST pulldown assays were performed in C2C12 myoblast cell lysates with subsequent 2D gelelectrophoresis and MALDI-TOF mass spectrometry analysis (Hassel et al., 2004). Data analyses identified cGKI as a BRII-tail-associated protein (Figure 8). Due to alternative splicing cGKI exists as two N-terminally different isoforms, cGKIα and cGKIβ. Both are expressed in C2C12 cells (Casteel et al., 2002), the identified peptides however did not allow a differentiation between both isoforms (Figure 9). To investigate the effects of cGKI in BMP signaling C2C12 cells and 293T cells, both BMP responsive, were used in the experiments. To discriminate between cGKIα and β, recombinant GST fusion proteins GST-BRII-SF, GST-BRII-tail and GST alone as bait in C2C12 cells overexpressing α- or β-isoform were used. After immunoblotting with α-cGKI antiserum the inventors found that both isoforms associate with BRII cytoplasmic domains (Figure 1A, 10). BRII-tail formed a strong complex with cGKIα and β (Figure 1A, lanes 4 and 9), but also BRII-SF interacted with both isoforms (Figure 1A, lanes 3 and 8). The full length receptor BRII-LF also bound both cGKIα and β as shown by co-immunoprecipitation from transfected 293T cells (Figure 1B). To confirm the data, the inventors performed confocal immunofluorescence microsopy to localize cGKIβ and BRII in cells. Living C2C12 cells stably expressing HA-tagged BRII-SF or BRII-LF were labeled at 4°C leading to receptor clustering at the cell surface (Figure 1C, left). Following cell fixation intracellular cGKIβ was visualized using a specific antibody (Figure 1C, middle). The merged images demonstrated co-localization of endogenous cGKIβ with HA-BRII-SF as well as HA-BRII-LF predominantly at the cell surface (Figure 1C, right). The soluble kinase is recruited to the membrane as a BRII interaction partner by co-patching of the overexpressed receptor.

In order to map the interaction site of cGKIβ on BRII, the inventors performed co-immunoprecipitation studies after co-expressing cGKIβ and different N-terminally HA-tagged truncation mutants (TCs) of BRII (Nohe et al., 2002) in 293T cells (Figure 1D, E). Immuno-precipitations using α-cGKIβ antiserum demonstrated that the BRII truncations (TC4-8) as well as both splice variants BRII-SF and BRII-LF associate with cGKIβ (Figure 1E). Only BRII-TC1 (Figure 1E, lane 1), the shortest deletion mutant lacking the receptor kinase and tail domain, did not bind cGKIβ. Consistent with this, C2C12 cells stably expressing HA-BRII-TC1 showed significantly reduced co-localization with endogenous cGKIβ at the cell surface when compared to wild type BRII (data not shown). Another intriguing observation was that, despite similar expression levels of both BRII isoforms, the interaction of cGKIβ with BRII-SF was weaker than with BRII-LF (Figure 1E, compare lanes 2 and 8), seen in several experiments (n>5). Therefore, BRII-LF offers two binding sites for cGKI, one in the kinase domain and one in the tail region of BRII.

cGKI exhibits a autoinhibitory/pseudo-substrate site at the N-terminus, which blocks the catalytic center in the inactive state. It mediates homodimerization via a leucine/isoleucine zipper motif, subcellular targeting and includes an autophosphorylation site involved in the raise of the basal activity of cGKI. The regulatory domain comprises two tandem cGMP binding sites. cGMP binding induces a conformational change whereby the catalytic center in the C-terminal kinase domain is released and substrates can be phosphorylated (Feil et al., 2005). Mapping the interaction site of BRII in the cGKI protein revealed that BRII-LF binds to the C-terminal half of cGKIβ including the kinase and the peptide binding domain, common to both cGKI isoforms (Figure 1F). To exclude that protein-protein interaction was driven by overexpression, the inventors analyzed endogenous protein complexes from C2C12 cells by co-immunoprecipitation. The inventors confirmed an interaction of endogenous cGKI and BRII (Figure 1G, lane 3). Furthermore, BRIa (Figure 1G, lane 2) and both TGFβ type II and type I receptors (TRII, TRI) interacted with cGKI (Figure 1G, lanes 4 and 5).

Taken together, the inventors determined interaction of both cGKI isoforms with BRII, whereas BRII offers presumably two cGKI binding sites. In addition, association of cGKI is not restricted to BRII, it includes also other receptors of the same family indicating that cGKI seems to have general affinity to BMP and TGFβ receptors via a common receptor site.

### cGKI phosphorylates BRII

To test whether serine/threonine kinase activities of BRII and cGKI are needed for the association, the inventors analyzed complex formation of wildtype cGKIβ or BRII-LF and the corresponding kinase inactive mutants cGKIβD516A and BRII-LF K230R in 293T cells by co-immunoprecipitation (Figure 2A). It was ruled out that neither BRII kinase activity (Figure 2A) nor cGKI kinase activity (Figure 2A) is necessary for the interaction of both proteins.

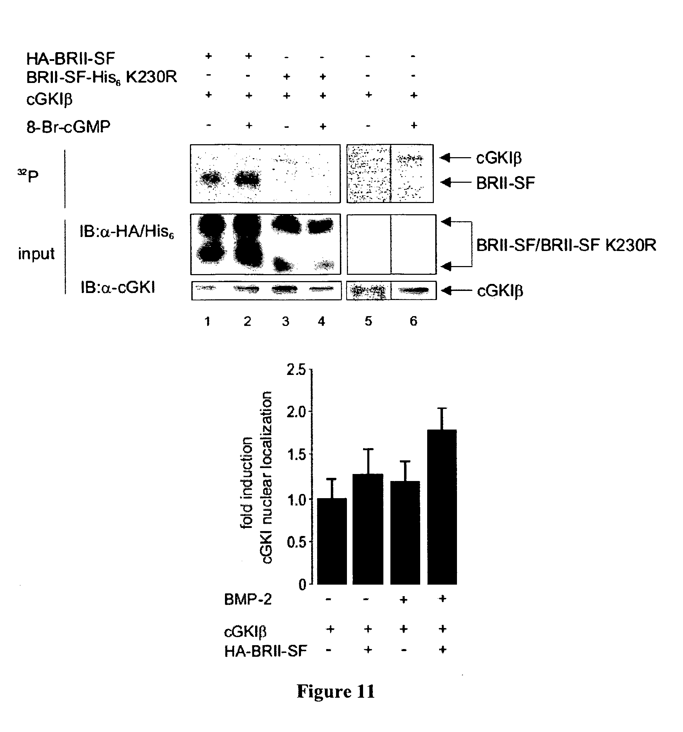

It was next examined whether cis or trans phosphorylation of cGKI or BRII was influenced by the association of both proteins. For this recombinant GST-BRII-tail and GST-BRII-SF (Figure 10) and cGKIα were subjected to *in vitro* phosphorylation using γ-³²P-ATP (Figure 2B). To activate cGKIα, the inventors added 8-Br-cGMP (Figure 2B). It was found that BRII-tail was phoshorylated by activated cGKIα (Figure 2B, lane 8). BRII-SF showed autophosphorylation which was unaffected by the absence or presence of cGKIα (Figure 2B, lanes 1-4). In this context, cGKIβ did not phosphorylate kinase deficient BRII-SF (Figure 11). In turn, cGKI was not phosphorylated by BRII kinase (Figure 2B, lanes 3 and 4, Figure 11). To investigate whether cGKI phosphorylates BRII *in vivo,* the inventors used C2C12 cells transfected with cGKIβ or empty vector. Cells were stimulated with 8-Br-cGMP for 30 min and lysed. Immunoprecipitation with an antibody directed towards BRII extracellular domain was followed by immunoblotting with an α-phosphopeptide antibody specific for substrates phosphorylated by arginine dependent kinases like cGKs (PKG) and the cAMP dependent kinase (PKA). The inventors found that upon overexpression of cGKIβ, BRII-LF is strongly phosphorylated (Figure 2C, upper, lane 4), while BRII-SF shows only weak phosphorylation with and without cGKIβ expression (Figure 2C, upper, lanes 3 and 4). It was observed that C2C12 cells express both BRII isoforms using immunoblot α-BRII (Figure 2C, middle), although BRII-LF was only detectable after accumulation via immunoprecipitation (Figure 2C, middle, lanes 3 and 4).

In sum, the activities of both serine/threonine kinases are not necessary for their interaction itself, while upon association cGKI phosphorylates BRII-tail *in vitro* and BRII-LF *in vivo*.

**cGKI is released from BRII and translocates into the nucleus after BMP-2 stimulation**

To investigate the fate of cGKIβ in response to activation of the BMP pathway, the inventors stimulated 293T cells transfected with HA-BRII-SF and cGKIβ constructs with BMP-2 for 5 to 60 min (Figure 3A). It was showed that upon serum starvation cGKIβ and BRII-SF do interact to a low extend (Figure 3A, longer exposure, lane 1), whereas ligand addition led to an increased interaction within 5 min persisting for 25 min (Figure 3A, upper, lanes 1-6) assuming that BMP-2 affects cGKI/BRII binding in the kinase region. Interestingly, prolonged stimulation entirely disrupted the interaction of BRII-SF with cGKIβ at 30 to 45 min (Figure 3A, lanes 7 and 8). Stimulation with BMP-2 for 60 min resulted in recovery of BRII-SF/cGKIβ interaction which differs in its rate between the experiments (Figure 3A, lane 9 and graph below). These dynamics in BRII/cGKI interactions are also reflected by using HA-BRII-LF instead of HA-BRII-SF (Figure 3B) although these complexes seem to be insensitive to serum starvation, i.e. do not need BMP-2 (Figure 3B, lane 1). Different binding modalities of BRII-SF and BRII-LF due to two binding sites in BRII-LF might be responsible for this. In general, that duration of serum starvation is very critical in studying the interaction of BRII and cGKI. The inventors conclude that the binding of BRII to cGKIβ underlies dynamic events including complete abrogation of interaction after ligand addition to recover again after about 1 hr (Figure 3A, B). To follow cGKI after the release from the receptor, the inventors performed immunofluorescence microscopy studies. Interestingly, addition of BMP-2 induced nuclear translocation of endogenous cGKI in C2C12 cells (Figure 3C, middle). Without ligand cGKI showed a pancellular distribution pointing towards a basal nuclear translocation or shuttling rate of cGKI in C2C12 cells. Furthermore, a redistribution of cGKI to the nucleus upon stimulation with 8-Br-cGMP in C2C12 cells (Figure 3C) (Gudi et al., 1998) was confirmed. According to this, it was observed that overexpressed cGKIβ undergoes nuclear translocation upon activation of BMP signaling in 293T cells (Figure 12).

These results show that BMP-2 stimulation triggers both dissociation of cGKI from the BMP receptors and nuclear translocation of cGKI in a distinct time frame.

### cGKI associates with Smads

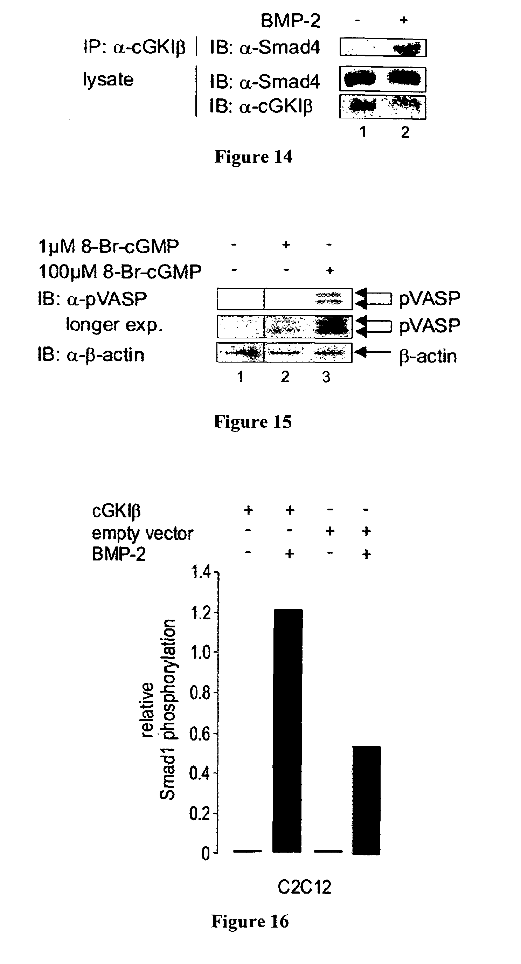

As demonstrated so far, cGKI is released from the cell surface receptor complexes after about 30 min. Moreover, BMP-2 triggers nuclear translocation of cGKI. Therefore it was asked whether cGKI associates with BMP R-Smads and/or co-Smad4 after dissociation from the receptors to undergo nuclear translocation. Binding studies in 293T cells using GST-fused cGKI proteins revealed that Smad1 interacts with full-length cGKIα and β isoforms (Figure 4A). Since it is known that activated Smad1/5/8 forms a complex with co-Smad4 before translocating into the nucleus (Shi and Massague, 2003), the inventors also investigated the interaction of cGKI with Smad4. Indeed, also Smad4 associates with both cGKI isoforms (Figure 4B). Furthermore, the inventors could map the interaction site for Smad1 and Smad4 to the C-terminal part of cGKI using truncation mutants of cGKI (Figure 4B). To further investigate these findings transfected 293T cells were stimulated with BMP-2 for 30 min or left untreated (Figure 4C, D). Following cGKIβ immunoprecipitation, the inventors found Smad1 (Figure 4C, a, lanes 1 and 2) and Smad5 (Figure 4C, a, lanes 3 and 4) to form complexes with cGKIβ already without ligand, but BMP-2 addition enhanced complex formation in both cases. According to this, the inventors observed that cGKIβ associated with phosphorylated Smad1 and Smad5 after reprobing the membrane with α-pSmad1/5/8 antibody (Figure 4C, b, lanes 2 and 4). It was striking that also binding of cGKIβ to Smad4 is BMP-2-regulated (Figure 4D, upper, lane 2). To confirm that cGKIβ associates preferentially with activated Smad complexes, the inventors examined endogenous cGKI/Smad complexes in different cellular compartments. Consistent with Figure 4C, it was determined that cGKI is associated with Smad1 in the cytoplasm already in the absence of ligand (Figure 4E, a, lane 5). Stimulation with BMP-2 leads to phosphorylation of Smad1 (Figure 4E, a and b, lanes 2, 4, 6 and 8) and to enhanced binding of phosphorylated Smad1 and cGKI (Figure 4E, a and b, lane 6). This association of cGKI and phosphorylated Smad1 was also detected in the nuclear fraction (Figure 4E, a and b, lane 8), albeit weaker than in the cytoplasm. The inventors performed the experiment in the absence of phosphatase inhibitors, which explains the relative lower amount of phospho-Smads in the nucleus compared to the cytoplasm; Smads get dephosphorylated in the nucleus. The interaction between endogenous cGKI and activated Smad complexes was confirmed also by co-immunoprecipitation in C2C12 whole cell lysates (Figure 13, 14).

To visualize the subcellular distribution of cGKI and Smad1, the inventors performed immunofluorescence microscopy using C2C12 cells stimulated with BMP-2 for different time periods (5 to 60 min). Without ligand the proteins showed a pancellular distribution. Following BMP stimulation both Smad1 and cGKI enrich in the nucleus with identical time kinetics (Figure 4F). Moreover, both proteins partly co-localized in the cytoplasm as well as in the nucleus of BMP-2- treated cells. Also Smad4 and cGKI show similar kinetics upon ligand application (Figure 4G). cGKI and R-Smad/Smad4 complexes revealed the strongest nuclear accumulation within 20 to 45 min after stimulation with BMP-2 to slowly come back to the initial status thereafter.

In sum, these results show that cGKI associates with R-Smads already in the absence of ligand whereas their binding is enhanced after BMP-2 stimulation. Within the activated Smad complexes cGKI also interacts with Smad4 to translocate with these complexes into the nucleus.

### cGKI enhances R-Smad phosphorylation

It was then investigated whether interaction of cGKI with R-Smads influence C-terminal Smad phosphorylation. The inventors expressed cGKIβ and Smad1 in the BMP-2 responsive cell line 293T. This resulted in phosphorylation of Smad1 under non-stimulated conditions which is enhanced after BMP-2/8-Br-cGMP co-stimulation (Figure 5A, a, lanes 1 and 2). In contrast kinase inactive cGKIβ D516A did not affect Smad1 phosphorylation (Figure 5A, a, lanes 3 and 4) suggesting that the kinase activity of cGKIβ promotes C-terminal phosphorylation of Smad1. This result was also obtained without 8-Br-cGMP co-stimulation (Figure 5B). The inventors monitored cGKIβ activity via VASP phosphorylation (serine 239) and demonstrated that overexpressed cGKIβ has a strong basal kinase activity (Figure 5A, c, lane 1) which can be enhanced by the addition of 1 µmol/l of 8-Br-cGMP (Figure 5A, c, lane 2). The higher band is caused by concomitant VASP phosphorylation on serine 157, a PKA site. Already low 8-Br-cGMP concentrations (1 µmol/l) were sufficient to induce weak cGKI-mediated VASP phosphorylation in C2C12 cells (Figure 15). The inventors observed the same enhancing effect of cGKI on endogenous Smad1/5/8 phosphorylation in C2C12 cells (Figure 16). To further analyze the function of cGKI in Smad phosphorylation, a shRNA construct specific for mouse *cGKI* (sh-cGKI) was designed. Validation of sh-cGKI for knockdown of endogenous cGKI was performed in C2C12 cells by immunoblotting (Figure 5C). Consistent with the Smad phosphorylation assays shown above, downregulation of endogenous cGKI via sh-cGKI in these cells resulted in a reduced Smad1/5/8 phosphorylation already without ligand (Figure 5D, compare lanes 2 and 4).

Taken together, these data show that cGKI promotes C-terminal phosphorylation of R-Smads, already before BMP activation of the receptor complexes.

### cGKI and Smad1 form complexes on the Id1 promoter in a BMP-2-dependent manner

Since the inventors have shown that cGKI interacts with Smads also inside the nucleus, it was next asked whether these complexes bind in common to promoter sites of BMP-2 target genes such as *Id1*. To investigate this, chromatin immunoprecipitation (ChIP) assays were performed with untreated C2C 12 cells or with cells either stimulated with BMP-2 or 8-Br-cGMP alone or with both ligands (Figure 6A). In unstimulated cells a small fraction of Smad1 was detectable at the *Id1* promoter (Figure 6A, a, lanes 4 and 5), whereas after BMP-2 stimulation a 5-fold stronger association of both Smad1 (Lopez-Rovira et al., 2002) and cGKI with the *Id1* promoter was observed (Figure 6A, b, lanes 4 and 5). Co-stimulation with 8-Br-cGMP or stimulation with 8-Br-cGMP alone did not affect the binding of Smad1 and cGKI pointing to that cGMP does neither induce the binding nor alter the binding strength of cGKI/Smad complexes to the promoter or its assembly kinetics (Figure 6A, c, d). On the other hand cGKI associates with the promoter after BMP-2 stimulation in the absence of 8-Br-cGMP which indicates that recruitment of cGKI to the *Id1* promoter is an important BMP-2-induced process. The inventors observed the same result in 293T cells (data not shown). To prove that Smad1 and cGKI form complexes at the *Id1* promoter, two-step ChIP experiments were carried out (Figure 6B). Indeed in experiments using an α-Smad1 antibody for the first ChIP and an α-cGKI antibody for the second ChIP, association of Smad1/cGKI complexes with the *Id1* promoter was detectable (Figure 6B, lanes 6 and 7). Strong binding of Smad1/cGKI complexes to the *Id1* promoter occurred in BMP-2 and BMP-2/8-Br-cGMP treated cells (Figure 6B, b and c, lanes 6 and 7). When IgG was used for the second ChIP, no co-localization could be observed (Figure 6B, lane 4). These results were confirmed in an assay using an inverted order of the antibodies (Figure 6B, lanes 5 and 7).

These results suggest that cGKI not only translocates with Smads into the nucleus but also binds with Smad1 to the *Id1* promoter indicating a regulatory role for cGKI in transcription activation.

### TFII-I co-localizes with Smad1 and cGKI on the Id1 promoter after BMP-2 stimulation

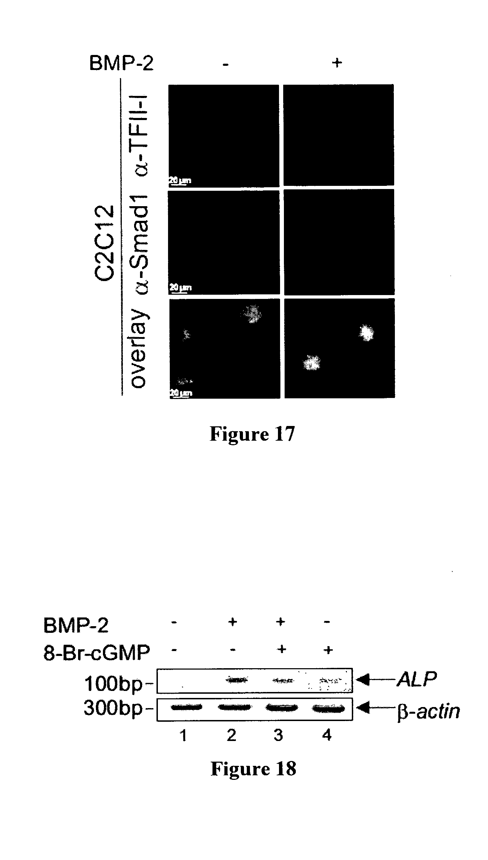

cGKIβ was shown to interact physically with the transcription factor TFII-I and to phosphorylate TFII-I leading to increased transactivation potential of TFII-I (Casteel et al., 2002). To investigate whether TFII-I is associated with cGKI/Smad complexes at the *Id1* promoter, ChIP and two-step ChIP experiments were performed. Indeed, TFII-I bound together with Smad1 to *Id1* promoter sites in BMP-2 and BMP-2/8-Br-cGMP-treated C2C12 cells (Figure 6C, b and c, lanes 5, 7 and 9) but not in unstimulated cells (Figure 6C, a). From these data the inventors conclude that cGKI and TFII-I in a BMP-2-dependent manner associate with Smad1 at the *Id1* promoter to form ternary transcription complexes. Consistent with our results shown in the ChIP assay, we demonstrated complex formation of endogenous TFII-I and cGKI, Smad1 and Smad4 by co-immunoprecipitation in C2C12 cells (Figure 6D). The double band seen for TFII-I represents its two splice forms, β and Δ (Hakre et al., 2006), detected here as a double band (Figure 6D, upper, lane 1). Interestingly, TFII-I molecules with a higher molecular weight were also pulled down with Smad1 and 4 (Figure 6D, upper, lanes 3 and 4) suggesting protein modifications occurring within TFII-I/Smad complexes or emerging of these interactions only after modification of TFII-I. Consistent with the ChIP assay in Figure 6C, Smad1/TFII-I binding was induced by BMP-2 in 293T cells (Figure 6E). Isoform-specific conformation as well as serum starvation, respectively growth factor stimulation regulate the subcellular localization of TFII-I (Hakre et al., 2006). For the C2C12 cell system, the inventors determined by immunofluorescence microscopy with a pan-TFII-I antibody that TFII-I is predominantly defined to the nucleus with or without BMP-2 stimulation and co-localizes with Smad1 in the nucleus after BMP-2 stimulation (Figure 17).

These data led us assume that TFII-I in concert with Smads and cGKI is a regulator of BMP signaling at the level of the target gene which joins the Smad complexes in the nucleus.

### cGKI enhances Smad-mediated transcription activation

To investigate the functional role of cGKI in BMP-2 triggered Smad signaling the inventors analyzed the effect of cGKI on the expression of Smad-dependent BMP-2 target genes in continuation of our results described before. Using a *BMP response element* (*BRE* from *Id1* promoter) luciferase reporter gene assay (Korchynskyi and ten Dijke, 2002), the inventors showed that both cGKIα and β stimulate the *BRE* reporter in C2C12 cells (Figure 7A, B). According to the Smad phosphorylation assays in Figure 5, wildtype cGKI increased *BRE* reporter activity even in the absence of BMP-2, while the kinase inactive mutant cGKIβ D516A failed to do so (Figure 7A, B). Similar results were obtained by co-expression of BRII-SF (Figure 7H) or BRII-LF (data not shown). Downregulation of endogenous cGKI after transfecting sh-cGKI clearly reduced *BRE* reporter gene response upon ligand stimulation to less than 50% when compared to control cells (Figure 7C). In this context the effect of cGKI on the induction of the endogenous BMP-2 target gene *Id1* was analyzed by RT-PCR (Ogata et al., 1993). According to the *BRE* reporter data it was found that *Id1* was upregulated upon cGKI expression already in the absence of ligand (data not shown). In spite evidences for that overexpressed cGKI has a strong basal, cGMP-independent kinase activity which is sufficient for phosphorylating and regulating its targets, the inventors checked the BRE response upon stimulation of endogenous cGKI with 8-Br-cGMP. Stimulation and co-stimulation with 1 µmol/l or 100 µmol/l 8-Br-cGMP did not affect the *BRE* reporter gene activity, while it was strongly induced by BMP-2 alone (Figure 7D). The inventors also analyzed the effect of cGMP treatment on the induction of *Id1* mRNA via RT-PCR and surprisingly found that stimulation with 8-Br-cGMP in C2C12 cells resulted in a potentiation of the BMP-2-induced increase in *Id1* transcription (Figure 7E, lane 3). While BMP-2 led to a more than 2-fold induction of *Id1*, BMP-2 together with 8-Br-cGMP resulted in 5-fold induction (Figure 7E). These results indicate that the cGMP/cGKI pathway influences the artificial minimal promoter and the endogenous *Id1* promoter differently.

The observations that TFII-I interacted with Smad1 and formed ternary complexes with Smad1 and cGKI at the *Id1* promoter suggested, that TFII-I also regulates BMP-2 signaling. To test this, the effect of TFII-I on *BRE* reporter gene activity in C2C12 cells was measured (Figure 7F). TFII-I enhances the reporter gene activity in BMP-2 stimulated cells (Figure 7F). Cells expressing TFII-I and cGKIα showed a stronger increase of *BRE* activity (Figure 7F) since cGKIα enhanced already the basal transcriptional response as shown in Figure 7A and B.

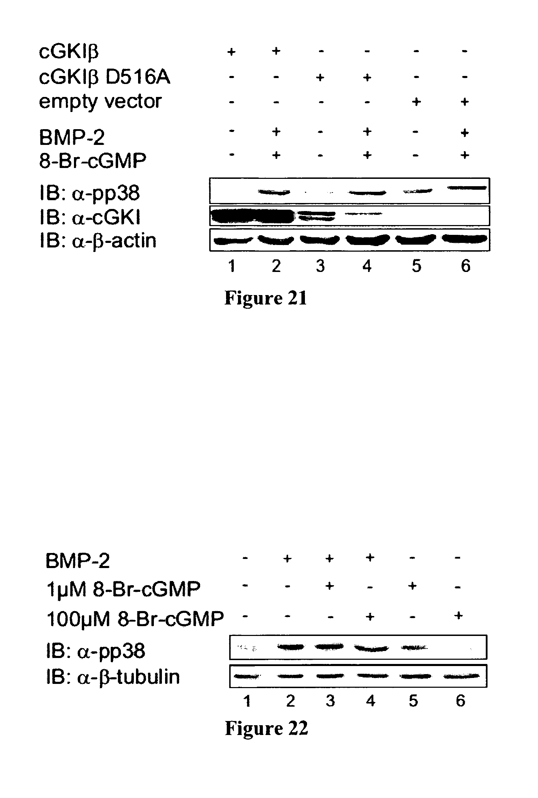

To investigate whether cGKI plays also a role in the regulation of other BMP-2 target genes, the induction of the osteogenic marker alkaline phosphatase (ALP) in C2C12 cells was analyzed. The inventors neither observed a BMP-2-dependent induction and activation of ALP (Figure 18, 19, 20) nor a BMP-2-dependent activation of MAPK p38 (Figure 21, 22), a key component of this pathway (Nohe et al., 2002), under the influence of cGMP/cGKI. Further investigation of BMP-2 target genes besides *Id1* and *ALP* revealed the interesting finding that *cGKI* transcription itself is induced upon BMP-2 stimulation. Four hours of stimulation with BMP-2 led to an upregulation of *cGKI* mRNA by a factor of 2 (Figure 7G). Notably, this is very similar to the fold increase of the transcription of the classical early target gene *Id1* (Figure 7E).

These experiments proof that the cGMP/cGKI pathway not only induces Smad1 phosphorylation but also enhances Smad dependent *Id1* gene expression with a strong indication that basal activity of cGKI is sufficient for promoting Smad signaling. Moreover, induction of *cGKI* by BMP-2 generates a feed forward mechanism to enhance BMP signaling.

Genetic studies in Pulmonary Arterial Hypertension (PAH) have revealed heterozygous germline mutations in BRII (Waite and Eng, 2003). PAH is characterized by remodeling of small pulmonary arteries by myofibroblasts and smooth muscle cell proliferation (Morell, 2006). Treatment with sildenafil, a PDE5 inhibitor, increases intracellular cGMP level in the affected tissue and thereby activates cGMP targets as cGKI. Therefore, the inventors tested the effect of cGKI on BMP signaling which is induced by the sporadic PAH mutant HA-BRII-LF Q657ins16 (Thomson et al., 2000). Interestingly, cGKI rescues defective BMP signaling (Figure 7H). The *BRE* activity is upregulated in cell overexpressing both cGKI and the PAH mutant cells when compared to cells expressing the mutant alone (Figure 7H). This suggests a role for cGKI in BRII signaling, which can stimulate BRII signaling and partially compensates the loss of a functional BRII-tail region due to a frameshift mutation at position Q657.

### Materials and Methods

The results of the experiments described below are shown in figures 1 to 23.

### Expression and purification of GST fused BRII and cGKI variants and GST pulldown

Recombinant protein expression and purification and identification of BRII associated proteins was done as previously described (Hassel et al., 2004). For characterization and mapping of protein interactions, C2C12 cells, C2C12 cells overexpressing cGKI or 293T cells overexpressing GST fused cGKI variants and BRII or Smad proteins were used. Analysis was done via SDS-PAGE and subsequent immunoblot.

### Immunoprecipitation

C2C12 cells or transfected 293T cells were lysed or starved for 3 hours (hrs) in DMEM/0.5 % FBS and stimulated with 10 nmol/l BMP-2 for 30 min or the indicated time periods in starvation medium before lysis. Cell lysis was carried out using Triton lysis buffer (1 % Triton X-100, 20 mmol/l Tris/HCl pH 7.5, 150 mmol/l NaCl, Complete^{®} EDTA free (Roche Diagnostics), 1 mmol/l PMSF) and immunoprecipitation was performed. Precipitates were washed extensively and were subjected to SDS-PAGE and immunoblot analysis.

### Immunofluorescence microscopy

For co-localization studies, C2C12 cells stably expressing N-terminally HA-tagged BRII-SF or BRII-LF were stained as described in (Gilboa et al., 2000). Analysis was done with 63-fold magnification at a Leica DMR (Leica) confocal microscope. To examine the protein localization C2C12 cells or transfected 293T cells were starved for 3 hrs and either stimulated with 10 or 20 nmol/l BMP-2 and/or 1 mmol/l 8-Br-cGMP for 30 min or for 5 to 60 min or left untreated. Indirect immunofluorescence was performed as described in (Bengtsson and Wilson, 2006). Cells were analyzed using fluorescence microscopy (63-fold magnification, Axiovert 200M, Zeiss).

### Nuclear-cytoplasmic fractionation

C2C12 cells were starved in DMEM/0.5 % FBS for 3 hrs, stimulated with 10 nmol/l BMP-2 for 30 min and collected in PBS. After centrifugation cells were resuspended in cytosolic lysis buffer (10 mmol/l Hepes pH 7.4, 2 mmol/l MgCl₂, 10 mmol/l KCl, 1 mmol/l EDTA, 1 mmol/l DTT, 10 mmol/l NaF, 0.1 mmol/l Na₃VO₄, Complete^{®} EDTA free) and incubated on ice. After addition of NP-40 (Sigma-Aldrich) to a final concentration of 0.5 %, cells were incubated on ice again. Vortexing and centrifugation separated cytoplasm from the nuclei and isolated nuclei were resuspended and lysed in Triton lysis buffer. Cleared cytoplasmic and nuclear lysates were subjected to immunoprecipitation.

### In vitro kinase assay

Immunopurified BRII variants and cGKIβ or recombinant BRII cytoplasmic domains, recombinant Smad1 and recombinant cGKIα (Promega) were subjected to *in vitro* kinase assay. Sepharose beads coupled proteins were supplemented with 25 µl kinase buffer (150 mmol/l NaCl, 20 mmol/l Hepes pH 7.4, 75 mmol/l MgCl₂, 500 µmol/l ATP, 1 mmol/l DTT) containing 25 µmol/l 8-Br-cGMP or not. Phosphorylation was initiated by addition of 1 µCi of (γ-³²P)ATP (Hartmann) and the precipitates were incubated at 30 °C. Proteins were separated on SDS-PAGE and transferred to nitrocellulose membrane. Phosphorylated proteins were detected using a phospho-imager (FLA-5000, Fujifilm) or X-ray films. Protein loading was determined by subsequent immunoblotting.

### In vivo kinase assay

Transfected C2C12 cells were starved for 3 hrs and stimulated with 1 µmol/l 8-Br-cGMP for 30 min. Cells were lysed in 1 % Triton-X 100 lysis buffer containing phosphatase inhibitors (5 mmol/l NaF, 2 mmol/l NaVO₄) and cleared lysates were subjected to immunoprecipitation for protein enrichment. After SDS-PAGE and Western blotting, the samples were probed with α-pPKA/PKG substrate antibody.

### Smad phosphorylation assay

Transfected 293T cells or C2C12 cells were starved in DMEM/0.5% FBS for 24 hrs and stimulated with 10 nmol/l BMP-2 with or without 1 µmol/l 8-Br-cGMP for 30 min. Cell lysis and immunoblotting was performed as described in (Hartung et al., 2006).

### cGKI knock down

C2C12 cells were transfected with sh-cGKI or sh-control. 48 hrs after transfection cells were lysed in Triton lysis buffer. Cleared lysates were subjected to immunoblotting.

### BRE luciferase reporter gene assay

C2C12 cells were transfected with p*BRE*₄-luc and pRL-TK and indicated constructs. Cells were treated with starvation medium for 5 hrs and stimulated with 1 nmol/l BMP-2 and/or 1 µmol/l or 100 µmol/l 8-Br-cGMP for 24 hrs. Luciferase activity was measured according to manufacturer's instructions using the Dual-Luciferase® Reporter Assay System (Promega) and a FB12 or Mithras LB 940 luminometer (Berthold). Expression control was examined by immunoblotting.

### BMP-2 target gene assay

C2C12 cells were starved in DMEM/0.5 % FBS and treated with 20 nmol/l BMP-2 and/or I µmol/l 8-Br-cGMP for 4 hrs. RNA extraction and reverse transcription were done as described in (Hartung et al., 2006). Analysis of mRNA amount was performed using *Id1*, *ALP, cGKI* and β*-actin* specific oligodeoxynucleotides.

### Chromatin immunoprecipitation

ChIP was performed as described previously (Weiske and Huber, 2006) with minor modifications. Briefly, C2C12 cells were grown to a confluence of 80-90% (10 cm dish). After stimulation with 10 nmol/l BMP and/or 1 µmol/l 8-Br-cGMP for 4 hrs, cells were washed with PBS, fixed with 2 mmol/l disuccinimidyl-glutarate, cross-linked using 1 % FA and the samples were subjected to immunoprecipitation with 2.5-5 µg of antibody. For two-step ChIP immunocomplexes of the first ChIP were eluted by adding 100 µl 10 mmol/l DTT (30 min at 37 °C) and diluted in ChIP dilution buffer followed by antibody incubation. ChIP and two-step ChIP were performed in the same way. For subsequent PCR analysis, extracted DNA was used as a template to amplify an *Id1* promoter fragment using specific oligodeoxynucleotides. PCR products were separated on 8 % PA gels and analyzed under UV light.

### Constructs

HA- or His₅-tagged BRII wildtype and mutant constructs are described in (Nohe et al., 2002), GST-fused BRII constructs in (Hassel et al., 2004) and Smad1, FLAG-tagged Smad5, Smad4 constructs in (Liu et al., 1996, Akiyoshi et al., 1999, Murakami et al., 2003, Caestecker et al., 1997). cGKIβ and cGKIβ D516A are described in Gudi et al., 1998, and Meinecke et al., 1994. N-terminally GST-fused cGKI constructs and TFII-I construct in (Casteel et al., 2005; Casteel et al., 2002) and *BRE*-reporter gene construct (p*BRE*₄-luc) in (Korchynskyi and ten Dijke, 2002). shRNA against cGKI (sh-cGKI, 5'-CACCGGGACGATGTTTCTAACA AACGAATTTGTTAGAAACATCGTCC-3', SEQ ID NO 3) and control shRNA (sh-control) in pENTR were obtained from H. Volkmer (NMI, Reutlingen).

### Antibodies

Immunoblotting, immunoprecipitation, immunostaining and chromatin immunoprecipitation were done with the following antibodies: α-HA antibody (Roche), α-cGKI antibody (Stress-gene), α-Smadl/5 antibody (Milipore), α-pSmad1/5/8 (C-terminal S*XS*) antibody (Cell Signaling Technology), α-p-PKA substrate (RRXS*/T*) antibody (Cell Signaling Technology), α-TFII-I antibody (BD Biosciences), α-β-actin antibody (Sigma-Aldrich), α-β-tubulin antibody (Sigma-Aldrich), α-P-p38 (pTGY*) antibody (Promega) and α-LaminA/C (clone IE4, McKeon). α-cGKIβ, α-pVASP (S*239) antibody, α-Smad1 antibody, α-Smad4 antibody, α-His₆ antibody and α-GST antibody were all purchased from Santa Cruz Biotechnology. α-BRIa, α-BRII, α-TRI and α-TRII antibodies were described earlier (Gilboa et al., 2000; Nohe et al., 2001; Rotzer et al., 2001). Peroxidase-conjugated secondary and fluorescent dye-coupled secondary antibodies (goat α-mouse IgG (H+L), Cy2-conjugated; mouse α-goat IgG (H+L), Cy3-conjugated; goat α-mouse IgG (H+L), conjugated to Alexa Fluor 594 or 488; or goat α-rabbit IgG (H+L), conjugated to Alexa Fluor 594) were purchased from Dianova, GE Healthcare and Invitrogen. S*, T*, Y* means phospho-serine, phospho-threonine or phospho-tyrosine, respectively.

### Cell culture and transfection

293T/HEK cells and C2C12 cells were obtained from the American Type Culture Collection (ATCC) and cultivated in Dulbecco's modified eagle medium (DMEM) supplemented with 10 % FBS. 293T cells were used for protein overexpression studies and transfected using polyethylenimine (PEI, Sigma-Aldrich) (Boussif et al., 1995). For transfection of C2C12 cells PEI or Lipofectamine^{™} (Invitrogen) was used according to manufacturer's instructions. Cells were used for continuative assays 24-48 hrs after transfection. C2C12 cells stably expressing BRII-HA were described by us earlier (Hassel et al., 2003).

### ALP activity assay

Transfected C2C12 cells or parental C2C12 cells were stimulated with 20 nmol/l BMP-2 and/or 1 or 100 µmol/l 8-Br-cGMP for 72 hrs in DMEM/2 % FBS, ALP activity was measured as described by us earlier (Nohe et al., 2002). Expression control of the pooled lysates was examined by immunoblot.

### p38 phosphorylation assay

C2C12 cells, transfected or not, were starved in DMEM/0.5 % FBS for 5 hrs and stimulated with 10 nmol/l BMP-2 and/or 1 or 100 µmol/l 8-Br-cGMP for 1 hr. Cells lysis and immunoblotting was done as described by (Hartung et al., 2006).

### Oligodeoxynucleotide sequences

All oligodeoxynucleotides were obtained from (Thermo, Fisher Scientific or Invitrogen). They are designed for the respective mouse mRNA sequence. The sequences (in 5' to 3' orientation) are: *Id1* (forward: AGGTGAAGCTCCTGCTCTACGA, SEQ ID NO 4; reverse: CAGGATCTCCACCTTGCTCACT, SEQ ID NO 5), ALP (forward: AATCGGAACAAC CTGACTGACC, SEQ ID NO 6; reverse: TCCTTCCACCAGCAAGAAGAA, SEQ ID NO *7), cGKI* (forward: GGGGTTCGTTTGAAGACTCA, SEQ ID NO 8; reverse: AGGATGAGATTCTCCGGCTT, SEQ ID NO 9) and β-*actin* (forward: CGGAACGCGTCA TTGCC, SEQ ID NO 10; reverse: ACCCACACTGTGCCCATCTA, SEQ ID NO 11). Template amplification in ChIP analysis was done with the following oligodeoxynucleotides detecting mouse *Id1* promoter (forward: GGAGCGGAGAATGCTCCAG, SEQ ID NO 12; reverse: GAAGGCCTCCGAGCAAGC, SEQ ID NO 13).

### References

1. Benezra, R., Davis, R. L., Lassar, A., Tapscott, S., Thayer, M., Lockshon, D., and Wein-traub, H. (1990). Id: a negative regulator of helix-loop-helix DNA binding proteins. Control of terminal myogenic differentiation. Ann N Y Acad Sci 599, 1-11.
2. Bois, P. R., Brochard, V. F., Salin-Cantegrel, A. V., Cleveland, J. L., and Grosveld, G. C. (2005). FoxO1a-cyclic GMP-dependent kinase I interactions orchestrate myoblast fusion. Mol Cell Biol 25, 7645-7656.
3. Boussif, O., Lezoualc'h, F., Zanta, M. A., Mergny, M. D., Scherman, D., Demeneix, B., and Behr, J. P. (1995). A versatile vector for gene and oligonucleotide transfer into cells in culture and in vivo: polyethylenimine. Proc Natl Acad Sci U S A 92, 7297-7301.
4. Canalis, E., Economides, A. N., and Gazzerro, E. (2003). Bone morphogenetic proteins, their antagonists, and the skeleton. Endocr Rev 24, 218-235.
5. Casteel, D. E., Boss, G. R., and Pilz, R. B. (2005). Identification of the interface between cGMP-dependent protein kinase Ibeta and its interaction partners TFII-I and IRAG reveals a common interaction motif. J Biol Chem 280, 38211-38218.
6. Casteel, D. E., Zhuang, S., Gudi, T., Tang, J., Vuica, M., Desiderio, S., and Pilz, R. B. (2002). cGMP-dependent protein kinase I beta physically and functionally interacts with the transcriptional regulator TFII-I. J Biol Chem 277, 32003-32014.
7. Chan, M. C., Nguyen, P. H., Davis, B. N., Ohoka, N., Hayashi, H., Du, K., Lagna, G., and Hata, A. (2007). A novel regulatory mechanism of the bone morphogenetic protein (BMP) signaling pathway Involving the carboxyl-terminal tail domain of BMP type II receptor. Mol Cell Biol 27, 5776-5789.
8. Chen, D., Zhao, M., and Mundy, G. R. (2004). Bone morphogenetic proteins. Growth Factors 22, 233-241.
9. Feil, R., Hofmann, F., and Kleppisch, T. (2005). Function of cGMP-dependent protein kinases in the nervous system. Rev Neurosci 16, 23-41.
10. Feng, X. H., and Derynck, R. (2005). Specificity and versatility in tgf-beta signaling through Smads. Annu Rev Cell Dev Biol 21, 659-693.
11. Foletta, V. C., Lim, M. A., Soosairajah, J., Kelly, A. P., Stanley, E. G., Shannon, M., He, W., Das, S., Massague, J., and Bernard, O. (2003). Direct signaling by the BMP type II receptor via the cytoskeletal regulator LIMK1. J Cell Biol 162, 1089-1098.
12. Gilboa, L., Nohe, A., Geissendorfer, T., Sebald, W., Henis, Y. I., and Knaus, P. (2000). Bone morphogenetic protein receptor complexes on the surface of live cells: a new oligomerization mode for serine/threonine kinase receptors. Mol Biol Cell 11, 1023-1035.
13. Harradine, K. A., and Akhurst, R. J. (2006). Mutations of TGFbeta signaling molecules in human disease. Ann Med 38, 403-414.
14. Hartung, A., Bitton-Worms, K., Rechtman, M. M., Wenzel, V., Boergermann, J. H., Hassel, S., Henis, Y. I., and Knaus, P. (2006). Different routes of bone morphogenic protein (BMP) receptor endocytosis influence BMP signaling. Mol Cell Biol 26, 7791-7805.
15. Hassel, S., Eichner, A., Yakymovych, M., Hellman, U., Knaus, P., and Souchelnytskyi, S. (2004). Proteins associated with type II bone morphogenetic protein receptor (BMPR-II) and identified by two-dimensional gel electrophoresis and mass spectrometry. Proteomics 4, 1346-1358.
16. Hassel, S., Schmitt, S., Hartung, A., Roth, M., Nohe, A., Petersen, N., Ehrlich, M., Henis, Y. I., Sebald, W., and Knaus, P. (2003). Initiation of Smad-dependent and Smad-independent signaling via distinct BMP-receptor complexes. J Bone Joint Surg Am 85-A Suppl 3, 44-51.
17. Hassel, S., Yakymovych, M., Hellman, U., Ronnstrand, L., Knaus, P., and Souchelnytskyi, S. (2006). Interaction and functional cooperation between the serine/threonine kinase bone morphogenetic protein type II receptor with the tyrosine kinase stem cell factor receptor. J Cell Physiol 206, 457-467.
18. Hemnes, A. R., and Champion, H. C. (2006). Sildenafil, a PDE5 inhibitor, in the treatment of pulmonary hypertension. Expert Rev Cardiovasc Ther 4, 293-300.
19. Hofmann, F., Feil, R., Kleppisch, T., and Schlossmann, J. (2006). Function of cGMP-dependent protein kinases as revealed by gene deletion. Physiol Rev 86, 1-23.
20. Hollnagel, A., Oehlmann, V., Heymer, J., Ruther, U., and Nordheim, A. (1999). Id genes are direct targets of bone morphogenetic protein induction in embryonic stem cells. J Biol Chem 274, 19838-19845.
21. Korchynskyi, O., and ten Dijke, P. (2002). Identification and functional characterization of distinct critically important bone morphogenetic protein-specific response elements in the Id1 promoter. J Biol Chem 277, 4883-4891.
22. Lee-Hoeflich, S. T., Causing, C. G., Podkowa, M., Zhao, X., Wrana, J. L., and Attisano, L. (2004). Activation of LIMK1 by binding to the BMP receptor, BMPRII, regulates BMP-dependent dendritogenesis. Embo J 23, 4792-4801.
23. Liu, F., Ventura, F., Doody, J., and Massague, J. (1995). Human type II receptor for bone morphogenic proteins (BMPs): extension of the two-kinase receptor model to the BMPs. Mol Cell Biol 15, 3479-3486.
24. Lohmann, S. M., and Walter, U. (2005). Tracking functions of cGMP-dependent protein kinases (cGK). Front Biosci 10, 1313-1328.
25. Lories, R. J., and Luyten, F. P. (2005). Bone morphogenetic protein signaling in joint homeostasis and disease. Cytokine Growth Factor Rev 16, 287-298.
26. Machado, R. D., Rudarakanchana, N., Atkinson, C., Flanagan, J. A., Harrison, R., Morrell, N. W., and Trembath, R. C. (2003). Functional interaction between BMPR-II and Tctex-1, a light chain of Dynein, is isoform-specific and disrupted by mutations underlying primary pulmonary hypertension. Hum Mol Genet 12, 3277-3286.
27. Nohe, A., Hassel, S., Ehrlich, M., Neubauer, F., Sebald, W., Henis, Y. I., and Knaus, P. (2002). The mode of bone morphogenetic protein (BMP) receptor oligomerization determines different BMP-2 signaling pathways. J Biol Chem 277, 5330-5338.
28. Pilz, R. B., and Broderick, K. E. (2005). Role of cyclic GMP in gene regulation. Front Biosci 10, 1239-1268.
29. Pilz, R. B., and Casteel, D. E. (2003). Regulation of gene expression by cyclic GMP. Circ Res 93, 1034-1046.
30. Rosenzweig, B. L., Imamura, T., Okadome, T., Cox, G. N., Yamashita, H., ten Dijke, P., Heldin, C. H., and Miyazono, K. (1995). Cloning and characterization of a human type II receptor for bone morphogenetic proteins. Proc Natl Acad Sci U S A 92, 7632-7636.
31. Roy, A. L. (2001). Biochemistry and biology of the inducible multifunctional transcription factor TFII-I. Gene 274, 1-13.
32. Rudarakanchana, N., Flanagan, J. A., Chen, H., Upton, P. D., Machado, R., Patel, D., Trembath, R. C., and Morrell, N. W. (2002). Functional analysis of bone morphogenetic protein type II receptor mutations underlying primary pulmonary hypertension. Hum Mol Genet 11, 1517-1525.
33. Sapkota, G., Alarcon, C., Spagnoli, F. M., Brivanlou, A. H., and Massague, J. (2007). Balancing BMP signaling through integrated inputs into the Smad1 linker. Mol Cell 25, 441-454.
34. Satow, R., Kurisaki, A., Chan, T. C., Hamazaki, T. S., and Asashima, M. (2006). Dullard promotes degradation and dephosphorylation of BMP receptors and is required for neural induction. Dev Cell 11, 763-774.
35. Schier, A. F., and Talbot, W. S. (2005). Molecular genetics of axis formation in zebrafish. Annu Rev Genet 39, 561-613.
36. Shi, Y., and Massague, J. (2003). Mechanisms of TGF-beta signaling from cell membrane to the nucleus. Cell 113, 685-700.
37. Stasyk, T., Dubrovska, A., Lomnytska, M., Yakymovych, I., Wernstedt, C., Heldin, C. H., Hellman, U., and Souchelnytskyi, S. (2005). Phosphoproteome profiling of transforming growth factor (TGF)-beta signaling: abrogation of TGFbeta1-dependent phosphorylation of transcription factor-II-I (TFII-I) enhances cooperation of TFII-I and Smad3 in transcription. Mol Biol Cell 16, 4765-4780.
38. Thomson, J. R., Machado, R. D., Pauciulo, M. W., Morgan, N. V., Humbert, M., Elliott, G. C., Ward, K., Yacoub, M., Mikhail, G., Rogers, P., et al. (2000). Sporadic primary pulmonary hypertension is associated with germline mutations of the gene encoding BMPR-II, a receptor member of the TGF-beta family. J Med Genet 37, 741-745.
39. Tournay, O., and Benezra, R. (1996). Transcription of the dominant-negative helix-loop-helix protein Id1 is regulated by a protein complex containing the immediate-early response gene Egr-1. Mol Cell Biol 16, 2418-2430.
40. Varga, A. C., and Wrana, J. L. (2005). The disparate role of BMP in stem cell biology. Oncogene 24, 5713-5721.
41. Waite, K. A., and Eng, C. (2003). From developmental disorder to heritable cancer: it's all in the BMP/TGF-beta family. Nat Rev Genet 4, 763-773.
42. Weiske, J., and Huber, O. (2006). The histidine triad protein Hint1 triggers apoptosis independent of its enzymatic activity. J Biol Chem 281, 27356-27366.
43. Wong, W. K., Knowles, J. A., and Morse, J. H. (2005). Bone morphogenetic protein receptor type II C-terminus interacts with c-Src: implication for a role in pulmonary arterial hypertension. Am J Respir Cell Mol Biol 33, 438-446.

## Claims

1. A method for restoring BMP-receptor signaling in a cell,
**characterized by**
increasing cGKI activity in a cell.

2. The method according to claim 1, **characterized by** overexpressing a polypeptide selected from the group consisting of:
(a) a polypeptide of SEQ ID NO 1 or SEQ ID NO 2;
(b) a polypeptide of aa 359 to 671 of SEQ ID NO 1 or of aa 374 to 686 of SEQ ID NO 2;
(c) a polypeptide comprising a portion of the polypeptide of (a) or (b) that exhibits cGKI function; and
(d) a polypeptide that is at least 80 % homologous to a polypeptide of (a) to (c).

3. The method according to claim 1 or 2, **characterized by** expressing a constitutively active form of cGKI in a cell.

4. The method according to claim 1 to 3, **characterized by** inactivating a protein that inhibits cGKI activity.

5. The method according to claims 1 to 4, wherein further a BMP-receptor ligand is provided to the cell.

6. Use of a method according to claims 1 to 5 for the treatment of a disease selected from the group consisting of pulmonary artery hypertension (PAH), cancer, fibrosis, bone diseases, and neurodegenerative diseases.

7. Use of cGKI for manufacturing a pharmaceutical composition for the treatment of a disease selected from the group consisting of pulmonary artery hypertension (PAH), cancer, fibrosis, bone diseases, and neurodegenerative diseases.

8. Use of a BMP receptor for screening for compounds having cGKI-like activity.

9. The use according to claim 8, wherein a BMP receptor protein is isolated from a cell under conditions that allow for the co-isolation of a protein that is functionally associated with the BMP receptor protein in the cell, and wherein the functionally associated protein is tested for cGKI activity.

10. Use of cGKI for screening of proteins associated with it.

11. The use according to claim 10, wherein the protein associated with cGKI is a receptor.

12. The use according to claim 10 or 11, wherein a cGKI protein is isolated from a cell under conditions that allow for the co-isolation of a protein that is functionally associated with the cGKI protein in the cell.

13. Use of cGKI for the transcriptional activation of a gene that comprises a BMP response element (BRE).

14. The use according to claim 13, wherein the gene further comprises a cGKI response element.


SEQUENCE LISTING

<110>  Freie Universität Berlin

<120>  A Method for Restoring BMP-Receptor Signaling in a Cell

<130>  FB19428

<160>  13

<170>  PatentIn version 3.3

<210>  1
<211>  671
<212>  PRT
<213>  Homo sapiens

<400>  1

Met Ser Glu Leu Glu Glu Asp Phe Ala Lys Ile Leu Met Leu Lys Glu
1               5                   10                  15

Glu Arg Ile Lys Glu Leu Glu Lys Arg Leu Ser Glu Lys Glu Glu Glu
            20                  25                  30

Ile Gln Glu Leu Lys Arg Lys Leu His Lys Cys Gln Ser Val Leu Pro
        35                  40                  45

Val Pro Ser Thr His Ile Gly Pro Arg Thr Thr Arg Ala Gln Gly Ile
    50                  55                  60

Ser Ala Glu Pro Gln Thr Tyr Arg Ser Phe His Asp Leu Arg Gln Ala
65                  70                  75                  80

Phe Arg Lys Phe Thr Lys Ser Glu Arg Ser Lys Asp Leu Ile Lys Glu
                85                  90                  95

Ala Ile Leu Asp Asn Asp Phe Met Lys Asn Leu Glu Leu Ser Gln Ile
            100                 105                 110

Gln Glu Ile Val Asp Cys Met Tyr Pro Val Glu Tyr Gly Lys Asp Ser
        115                 120                 125

Cys Ile Ile Lys Glu Gly Asp Val Gly Ser Leu Val Tyr Val Met Glu
    130                 135                 140

Asp Gly Lys Val Glu Val Thr Lys Glu Gly Val Lys Leu Cys Thr Met
145                 150                 155                 160

Gly Pro Gly Lys Val Phe Gly Glu Leu Ala Ile Leu Tyr Asn Cys Thr
                165                 170                 175

Arg Thr Ala Thr Val Lys Thr Leu Val Asn Val Lys Leu Trp Ala Ile
            180                 185                 190

Asp Arg Gln Cys Phe Gln Thr Ile Met Met Arg Thr Gly Leu Ile Lys
        195                 200                 205

His Thr Glu Tyr Met Glu Phe Leu Lys Ser Val Pro Thr Phe Gln Ser
    210                 215                 220

Leu Pro Glu Glu Ile Leu Ser Lys Leu Ala Asp Val Leu Glu Glu Thr
225                 230                 235                 240

His Tyr Glu Asn Gly Glu Tyr Ile Ile Arg Gln Gly Ala Arg Gly Asp
                245                 250                 255

Thr Phe Phe Ile Ile Ser Lys Gly Thr Val Asn Val Thr Arg Glu Asp
            260                 265                 270

Ser Pro Ser Glu Asp Pro Val Phe Leu Arg Thr Leu Gly Lys Gly Asp
        275                 280                 285

Trp Phe Gly Glu Lys Ala Leu Gln Gly Glu Asp Val Arg Thr Ala Asn
    290                 295                 300

Val Ile Ala Ala Glu Ala Val Thr Cys Leu Val Ile Asp Arg Asp Ser
305                 310                 315                 320

Phe Lys His Leu Ile Gly Gly Leu Asp Asp Val Ser Asn Lys Ala Tyr
                325                 330                 335

Glu Asp Ala Glu Ala Lys Ala Lys Tyr Glu Ala Glu Ala Ala Phe Phe
            340                 345                 350

Ala Asn Leu Lys Leu Ser Asp Phe Asn Ile Ile Asp Thr Leu Gly Val
        355                 360                 365

Gly Gly Phe Gly Arg Val Glu Leu Val Gln Leu Lys Ser Glu Glu Ser
    370                 375                 380

Lys Thr Phe Ala Met Lys Ile Leu Lys Lys Arg His Ile Val Asp Thr
385                 390                 395                 400

Arg Gln Gln Glu His Ile Arg Ser Glu Lys Gln Ile Met Gln Gly Ala
                405                 410                 415

His Ser Asp Phe Ile Val Arg Leu Tyr Arg Thr Phe Lys Asp Ser Lys
            420                 425                 430

Tyr Leu Tyr Met Leu Met Glu Ala Cys Leu Gly Gly Glu Leu Trp Thr
        435                 440                 445

Ile Leu Arg Asp Arg Gly Ser Phe Glu Asp Ser Thr Thr Arg Phe Tyr
    450                 455                 460

Thr Ala Cys Val Val Glu Ala Phe Ala Tyr Leu His Ser Lys Gly Ile
465                 470                 475                 480

Ile Tyr Arg Asp Leu Lys Pro Glu Asn Leu Ile Leu Asp His Arg Gly
                485                 490                 495

Tyr Ala Lys Leu Val Asp Phe Gly Phe Ala Lys Lys Ile Gly Phe Gly
            500                 505                 510

Lys Lys Thr Trp Thr Phe Cys Gly Thr Pro Glu Tyr Val Ala Pro Glu
        515                 520                 525

Ile Ile Leu Asn Lys Gly His Asp Ile Ser Ala Asp Tyr Trp Ser Leu
    530                 535                 540

Gly Ile Leu Met Tyr Glu Leu Leu Thr Gly Ser Pro Pro Phe Ser Gly
545                 550                 555                 560

Pro Asp Pro Met Lys Thr Tyr Asn Ile Ile Leu Arg Gly Ile Asp Met
                565                 570                 575

Ile Glu Phe Pro Lys Lys Ile Ala Lys Asn Ala Ala Asn Leu Ile Lys
            580                 585                 590

Lys Leu Cys Arg Asp Asn Pro Ser Glu Arg Leu Gly Asn Leu Lys Asn
        595                 600                 605

Gly Val Lys Asp Ile Gln Lys His Lys Trp Phe Glu Gly Phe Asn Trp
    610                 615                 620

Glu Gly Leu Arg Lys Gly Thr Leu Thr Pro Pro Ile Ile Pro Ser Val
625                 630                 635                 640

Ala Ser Pro Thr Asp Thr Ser Asn Phe Asp Ser Phe Pro Glu Asp Asn
                645                 650                 655

Asp Glu Pro Pro Pro Asp Asp Asn Ser Gly Trp Asp Ile Asp Phe
            660                 665                 670

<210>  2
<211>  686

<212>  PRT
<213>  Homo sapiens

<400>  2

<210> 3
<211> 47
<212> DNA
<213> Homo sapiens

<400> 3
caccgggacg atgtttctaa caaacgaatt tgttagaaac atcgtcc 47

<210> 4
<211> 22
<212> DNA

<213>  Mus musculus

<400>  4
aggtgaagct cctgctctac ga                                              22


<210>  5
<211>  22
<212>  DNA
<213>  Mus musculus

<400>  5
caggatctcc accttgctca ct                                              22


<210>  6
<211>  22
<212>  DNA
<213>  Mus musculus

<400>  6
aatcggaaca acctgactga cc                                              22


<210>  7
<211>  21
<212>  DNA
<213>  Mus musculus

<400>  7
tccttccacc agcaagaaga a                                               21


<210>  8
<211>  20
<212>  DNA
<213>  Mus musculus

<400>  8
ggggttcgtt tgaagactca                                                 20


<210>  9
<211>  20
<212>  DNA
<213>  Mus musculus

<400>  9
aggatgagat tctccggctt                                                 20


<210>  10
<211>  17
<212>  DNA
<213>  Mus musculus

<400>  10
cggaacgcgt cattgcc                                                    17


<210>  11
<211>  20
<212>  DNA

<213>  Mus musculus

<400>  11
acccacactg tgcccatcta                                                    20


<210>  12
<211>  19
<212>  DNA
<213>  Mus musculus

<400>  12
ggagcggaga atgctccag                                                     19


<210>  13
<211>  18
<212>  DNA
<213>  Mus musculus

<400>  13
gaaggcctcc gagcaagc                                                      18